# FASCICULE DE BREVET EUROPEEN

(11) **EP 0 712 414 B1**
(45) Date de publication et mention de la délivrance du brevet: **28.06.2006**
(21) Numéro de dépôt: 94924830.6
(22) Date de dépôt: 02.08.1994
(51) Int. Cl.: C12N 15/62, C07K 14/00

(54) **VECTEUR RECOMBINANT CONTENANT UNE SEQUENCE D'UN GENE DE LIPOPROTEINE POUR L'EXPRESSION DE SEQUENCES DE NUCLEOTIDES**
REKOMBINANTER VEKTOR MIT GENSEQUENZ VON LIPOPROTEINE ZUR NUKLEOTIDEFREQUENZEN EXPRESSION
RECOMBINANT VECTOR CONTAINING A LIPOPROTEIN GENE SEQUENCE FOR EXPRESSING NUCLEOTIDE SEQUENCES

(30) Priorité: 02.08.1993 FR 9309511
(43) Date de publication de la demande: 22.05.1996
(73) Titulaire: VRIJE UNIVERSITEIT BRUSSEL, 1050 Brussel (BE)
(72) Inventeur: Hamers, Raymond, 1640 Sint-Genesius-Rode (BE); Cornelis, Pierre, B- 1050 Brussel (BE)
(74) Mandataire: Desaix, Anne
(86) Numéro de dépôt international: PCT/EP1994/002550
(87) Numéro de publication internationale: WO 1995/004079

(56) Documents cités:
- WO-A-84/03519
- WO-A-93/07897
- DE-C- 4 122 598
- TRENDS IN BIOTECHNOLOGY., vol.11, no.1, Janvier 1993, CAMBRIDGE GB pages 6 - 10 G.GEORGIOU ET AL.
- PROTEIN ENGINEERING, vol.6, no.4, Juin 1993, OXFORD GB pages 449 - 454 M.L.LAUKKANEN ET AL.
- BIOLOGICAL ABSTRACTS, vol. 88 Philadelphia, PA, US; abstract no. 084626, DUCHENE M ET AL 'PSEUDOMONAS-AERUGINOSA OUTER MEMBRANE LIPOPROTEIN I GENE MOLECULAR CLONING SEQUENCE AND EXPRESSION IN ESCHERICHIA-COLI' & J BACTERIOL, 171 (8). 1989. 4130-4137.

## Description

L'invention est relative à un vecteur recombinant contenant une séquence d'un gène de lipoprotéine de structure, pour l'expression de séquences de nucléotides.

L'un des buts de l'invention est de proposer de nouveaux moyens pour préparer par les techniques du génie génétique, des protéines, polypeptides ou peptides, sous la forme de produits (éventuellement Ce produits de fusion) susceptibles d'être exposés au niveau de la membrane externe de la cellule qui les produit. En d'autres termes, l'invention fournit des vecteurs pour l'expression par des cellules recombinantes, de protéines extracellulaires ancrées dans la membrane de ces cellules.

On connait dans l'état de la technique, différents vecteurs permettant d'exprimer dans des hôtes cellulaires recombinants, des séquences d'acides aminés et permettant le cas échéant l'exposition de ces protéines à la surface de l'hôte cellulaire. On a par exemple eu recours, dans l'état de la technique, à des plasmides ou à des phages, pour exprimer des polypeptides dans des bactéries Gram-négatives et exposer à leur surface.

Ainsi Francisco J.A. et al (Bio/Technology vol. 11, Avril 1993, p. 491-495) ont exprimé dans E.coli, une exoglucanase sous la forme d'une protéine hybride (protéine de fusion) avec le domaine transmembranaire de la protéine OmpA naturellement contenue dans la membrane externe de E.coli, elle-même fusionnée avec les neuf premiers acides aminés de la lipoprotéine majeure de E.coli (encore appelée lipoprotéine (Lpp) de Braun, exprimée au niveau de la membrane externe). Selon Francisco et al, la protéine de fusion ainsi obtenue est ancrée à la surface de la membrane externe de E.coli.

Francisco et al ont eu recours à un produit de fusion faisant appel à la protéine OMPA qui permet au sein de cette construction hybride, l'exposition de l'exoglucanase à la surface de E. coli.

D'autres auteurs, Fuchs P. et al (Bio/Technology vol. 9, Décembre 1991, p. 1369-1372) ont décrit l'expression dans E.coli d'une protéine de fusion formée par les domaines variables des chaines lourdes et des chaines légères d'anticorps, fusionnée avec la lipoprotéine associée aux peptidoglycanes (PAL), de E.coli. Pour obtenir un produit de fusion accessible à la surface des cellules de E.coli, une séquence d'exportation d'une pectine lyase a été utilisée et la cystéine N-terminale de la PAL a été transformée en glycine, empêchant ainsi l'attachement des lipides de la membrane cellulaire externe à la cystéine normalement présente dans la PAL.

Les lipoprotéines mises en oeuvre par Fuchs et al selon la publication précitée, sont des lipoprotéines appartenant à la membrane cytoplasmique de la cellule.

Les inventeurs de la présente demande se sont intéressés à d'autres lipoprotéines, notamment à des lipoprotéines de structure analogues à la lipoprotéine de Braun de E. coli dans la mesure où elles ont la capacité de s'attacher à la membrane cellulaire externe d'une part et dans certains cas aux peptidoglycannes d'autre part. Ces lipoprotéines ont été utilisées dans le cadre de l'invention pour préparer des vecteurs recombinants destinés notamment à l'expression et à l'exportation à la surface de la membrane externe de cellules recombinantes, de séquences d'acides aminés.

De préférence la lipoprotéine mise en oeuvre n'est pas une lipoprotéine de E. coli.

L'invention a pour objet un vecteur recombinant pour le clonage et/ou l'expression et/ou le transfert dans un hôte cellulaire d'une séquence de nucléotides hétérologue caractérisé en ce qu'il comprend, en un site non essentiel pour sa réplication, le gène codant pour une lipoprotéine différente des lipoprotéines de E. coli, ou une partie de ce gène contenant les éléments nécessaires au contrôle de l'expression de cette lipoprotéine et à son exposition à la surface de la membrane cellulaire externe de l'hôte, de façon telle que la séquence de nucléotides hétérologue est susceptible d'être introduite dans le gène ou la partie du gène codant pour la lipoprotéine, dans des conditions permettant l'expression de cette séquence hétérologue et l'exposition du polypeptide obtenu à la surface de l'hôte cellulaire.

Par l'expresssion "séquence de nucléotides hétérologue", on entend toute séquence de nucléotides qui n'est pas naturellement contenue dans le vecteur non recombiné utilisé pour préparer les vecteurs de l'invention.

La taille de la séquence de nucléotides peut varier considérablement, de quelques nucléotides (par exemple 9) à plusieurs centaines de nucléotides, par exemple jusqu'à 1 kb ou 2 kb.

En d'autres termes, la séquence de nucléotides contenue dans le vecteur peut coder pour des protéines ayant un poids moléculaire pouvant varier jusqu'à une centaine de kilodaltons.

La séquence de nucléotides hétérologue est de préférence introduite au niveau de la partie N-terminale de la protéine Lpp mature, par exemple 3 à 4 acides aminés suivant l'extrémité N-terminale.

De préférence la lipoprotéine de structure dont il est question ci-dessus est une lipoprotéine de structure d'une bactérie.

Le vecteur recombinant de l'invention contient une lipoprotéine dite lipoprotéine de structure, par opposition à la lipoprotéin PAL de E. coli.

De préférence, il s'agit d'une lipoprotéine de bactérie Gram-négative.

Le gène codant pour cette lipoprotéine est de préférence introduit dans sa totalité dans le vecteur. Cependant, on peut également utiliser uniquement une ou plusieurs parties de ce gène dès lors que les éléments nécessaires au contrôle de l'expression de la lipoprotéine et à son exposition à la surface de la membrane cellulaire externe de l'hôte cellulaire modifié par le vecteur de l'invention, sont présents dans la construction. En particulier, on aura recours à une partie du gène codant pour une lipoprotéine qui contient ce que l'on appelle la "lipoprotéin box" (Milton J. et al., CRC Press (1993) International Standard Book Number 0-8493-6740-9, Chapter 6, p.163-181). Cette région correspond à une région carboxy-terminale présente dans la séquence signal de la lipoprotéine.

Le gène ou la partie du gène codant pour une lipoprotéine est introduit dans le vecteur de l'invention de façon telle que ce gène ou cette partie de gène peut en outre contenir une séquence de nucléotides hétérologue présente dans la lipoprotéine de façon telle qu'elle est exprimée et exposée à la surface de la membrane cellulaire d'un hôte chez lequel on aura préalablement introduit le vecteur recombinant.

Selon un premier mode de réalisation de l'invention, le vecteur recombinant est caractérisé en ce que le gène codant pour la lipoprotéine de structure ou la partie de ce gène, est modifié(e) par l'insertion d'une séquence de nucléotides hétérologue.

Cette insertion peut donc être réalisée après l'introduction dans un vecteur donné du gène de la lipoprotéine ou d'une partie de ce gène.

On peut également selon une autre variante de réalisation, introduire une séquence de nucléotides hétérologue dans le gène de la lipoprotéine ou une partie de ce gène et introduire ensuite cette séquence recombinante dans le vecteur choisi pour construire un vecteur recombinant selon l'invention.

Avantageusement, le vecteur recombinant est un plasmide.

Selon une première variante de réalisation de l'invention, la lipoprotéine dont on utilise le gène ou une partie du gène est une protéine hétérologue par rapport à l'hôte cellulaire contenant le vecteur.

En d'autres termes, cette lipoprotéine spécifique n'est pas naturellement présente dans l'hôte cellulaire choisi pour l'expression et/ou le clonage du vecteur recombinant de l'invention. Il n'est cependant pas exclu que l'hôte cellulaire choisi exprime naturellement une autre lipoprotéine.

De préférence le gène codant pour la lipoprotéine ou une partie de ce gène utilisée pour préparer le vecteur de l'invention est le gène codant pour la lipoprotéine majeure de la membrane externe d'une bactérie du genre Pseudomonas. De façon préférée, la bactérie est une bactérie de type Pseudomonas aeruginosa.

D'autres lipoprotéines peuvent également être mises en oeuvre et à cet égard, on citera les lipoprotéines de Braun et en particulier celle de S. marcescens (Nakamurak et al. PNAS, USA 77 (1980) 1369), E. amylovora (Yamagata H. et al., J. Biol. Chem., 256_(1981) 2194), M. morganii (Huang Y. X. et al., J. Biol. Chem., 258 (1983) 8139), P. mirabilis (Ching G. et al., J. Biol. Chem., 261 (1986) 4600) ; il peut également s'agir d'autres protéines de bactéries gram-négatives et en particulier les suivantes : Pullulanase de K. pneumoniae (Chapon C. et al., "Structure of two divergent promoters in front of the gene encoding pullulanase in Klebsiella pneumoniae and positively regulated", J. Bacteriol., 164 (1985) 639), K. aerogenes (Katsuragi N. et al., "Entire nucleotide séquence of pullulanase gène of K. aerogenes WVO", J. Bacteriol., 169 (1987) 2301), Pul S de K. pneumoniae (d'Enfert C. et al., "K. pneumoniae puIS gene encodes an outer membrane lipoprotein required for pullulanase secretion", J. Bacteriol., 171 (1989) 3673), chitobiase de V. harveyi (Soto-Gil R. et al. "Diacetylchitobiase of V. harveyi", J. Biol. Chem., 264 (1989) 14778), β-1,4-endoglucanase de P. solanacearum (Huang J. et al. "Excretion of the eft gène product of P. solanacearum", J. Bacteriol., 171 (1989) 3767), Pal et Pcp de H. influenzae (Deich R. "Cloning of genes encoding a 15,000-Dalton peptidoglycan-associated outer membrane lipoprotein and an antigenically related 15,000-Dalton protein from H. influenzae", J. Bacteriol., 170 (1988) 489), sous unité cytochrome de Rps. Viridis (Weyer K., "The cytochrome subunit of the photosynthetic reaction center from Rps. viridis is a lipoprotein", Biochemistry, 26 (1987) 2909).

En vue de faciliter l'insertion ou le contrôle de l'insertion de la séquence de nucléotides hétérologue dans le gène ou la partie de gène de la lipoprotéine, un ou plusieurs sites de restriction peuvent être introduits dans ce gène ou cette partie de gène, par l'intermédiaire par exemple d'un multi-adaptateur (polylinker).

De préférence, les sites de restriction du multi-adaptateur sont uniques par rapport aux sites présents naturellement dans le vecteur avant recombinaison et par rapport aux sites présents dans le gène ou la partie de gène codant pour la lipoprotéine.

Selon la nature du vecteur choisi pour préparer le vecteur recombinant de l'invention et en particulier du promoteur contrôlant l'expression de la séquence, l'expression de la séquence de nucléotides hétérologue peut être constitutive ou au contraire inductible par exemple par les métaux et notamment par le zinc ou par des inducteurs tels que l'IPTG.

De plus un vecteur selon l'invention peut être présent dans les cellules recombinantes de façon permanente ou transitoire. A cet égard, la séquence hétérologue peut être maintenue dans la cellule hôte par l'intermédiaire du vecteur de l'invention, sous la forme d'un plasmide. Elle peut selon une variante être au contraire intégrée dans les chromosomes de la cellule hôte.

La présente demande a en particulier pour objet le plasmide pLPI34 déposé à la Collection BCCM (Belgian Coordinated Collections of Microorganisms) au LMBP (Laboratorium voor Moleculaire Biologie-Plasmidencollectie) le 27 juillet 1993 sous le n° LMBP2916.

D'autres vecteurs particulièrement préférés pour la réalisation de l'invention sont les vecteurs pLPI35 et pLPI36 modifiés par rapport aux vecteurs pLPI34, par l'insertion d'un polylinker.

D'autres vecteurs particulièrement préférés sont les vecteurs -pVUB-1 et pVUB-2.

Le vecteur recombinant selon l'invention constitue un moyen pour préparer dans un hôte cellulaire, des antigènes ou de façon plus générale des séquences d'acides aminés de toutes origines et de toutes structures ou fonctions.

La capacité du vecteur recombinant de l'invention à exprimer sous forme exposée à la membrane cellulaire externe de l'hôte cellulaire, la séquence d'acides aminés codée par la séquence de nucléotides hétérologue permet aisément de purifier la séquence d'acides aminés ainsi obtenue. Par ailleurs il est possible d'utiliser directement les cellules recombinantes, notamment les bactéries recombinantes, exprimant à leur surface la séquence d'acides aminés codée par la séquence hétérologue, sans avoir recours à la lyse préalable des cellules hôtes. Ceci est notamment intéressant dans le cas d'utilisation des cellules hôtes dans des vaccins vivants.

En particulier, le vecteur recombinant de l'invention peut être mis en oeuvre pour exprimer des séquences d'acides aminés contenant un ou plusieurs déterminants antigéniques ou un ou plusieurs haptènes.

De façon générale un tel vecteur permet l'expression de tout peptide, polypeptide ou de toute protéine recombinante. Ce peptide, polypeptide ou cette protéine sera exprimé sous la forme d'un produit de fusion avec la lipoprotéine ou une partie de la lipoprotéine dont la séquence codante est présente dans le vecteur recombinant.

Avantageusement, on peut utiliser le vecteur recombinant de l'invention pour exprimer une séquence de nucléotides hétérologue codant pour un épitope de type T d'un antigène déterminé d'un agent pathogène. Ces agents pathogènes peuvent être des bactéries, des virus, des parasites ou de façon intéressante tout agent contre lequel on recherche une activité vaccinante ou thérapeutique.

Avantageusement, la séquence nucléotidique hétérologue contenue dans le vecteur, code pour un épitope T d'un antigène donné et pour un épitope B de ce même antigène ou d'un autre antigène d'un organisme pathogène contre lequel on recherche une immunisation.

Dans la perspective d'une des applications de l'invention, c'est-à-dire la préparation de compositions immunogènes, les peptides, polypeptides ou protéines de fusion (appelés plus généralement protéines de fusion) comportent un ou plusieurs antigènes et/ou un ou plusieurs déterminants antigéniques d'un agent pathogène tel qu'un parasite, une bactérie ou un virus par exemple, notamment d'un virus tel que celui de l'hépatite A, B ou C, d'un virus HIV et par exemple des antigènes d'enveloppe de HIV.

Des exemples non limitatifs sont donnés dans la partie expérimentale qui suit.

Selon un autre mode de réalisation particulier de l'invention, le vecteur contient une séquence de nucléotides hétérologue codant pour une séquence d'acides aminés d'un anticorps. Une telle séquence peut être une séquence d'acides aminés d'une chaîne lourde ou d'une chaîne légère d'un anticorps à 4 chaînes ou encore avantageusement une séquence codant pour un anticorps non pathologique constitué exclusivement de chaînes lourdes. A cet égard, référence est faite à la publication de Hamers et al., Nature, Vol. 363, 3 juin 1993, p.446-448 décrivant l'obtention et la caractérisation de tels anticorps.

L'invention a également pour objet une cellule hôte recombinante caractérisée en ce qu'elle est modifiée par un vecteur recombinant selon l'invention. Selon un mode de réalisation avantageux de l'invention, cette cellule hôte n'exprime pas naturellement la lipoprotéine codée par le gène ou la partie de gène contenue dans le vecteur.

Une telle cellule hôte est par exemple une bactérie gram-négative et notamment une souche de E. coli ou une souche de Alcaligenes eutrophus.

Le vecteur modifié par des séquences nucléotidiques codant pour un anticorps peut être par exemple introduit dans une cellule hôte, notamment dans une bactérie capable alors de produire des anticorps exposés à la surface de l'hôte, lesdites cellules recombinantes pouvant ainsi être utilisées par exemple comme adsorbant pour la purification d'antigènes.

L'invention a également pour objet une protéine recombinante caractérisée en ce qu'il s'agit du produit d'expression par un hôte cellulaire défini ci-dessus, du gène ou d'une partie du gène d'une lipoprotéine et d'une séquence de nucléotides hétérologue.

Une protéine recombinante de l'invention est une protéine de fusion entre une lipoprotéine choisie parmi les lipoprotéines différentes de celles de E. coli et/ou une partie d'une telle lipoprotéine et une séquence déterminée d'acides aminés.

Cette protéine recombinante, lorsqu'elle contient un ou plusieurs déterminants antigèniques, a la propriété de permettre in vivo la production d'anticorps dirigés contre le déterminant antigènique codé par la séquence de nucléotides hétérologues définie plus haut sans que cette production soit gênée par la présence de la molécule porteuse, en l'occurence la lipoprotéine.

En outre, les résultats obtenus démontrent que la protéine de fusion réalisée permet in vivo la production d'anticorps contre l'antigène codé par la séquence hétérologue sans qu'il soit nécessaire d'ajouter un adjuvant à la composition administrée.
L'invention a donc pour objet des compositions immunogènes comprenant à titre de principe actif, des protéines recombinantes telles que définies précédemment, portant un ou plusieurs déterminants antigéniques contre lesquels on cherche à obtenir des anticorps.

D'autres compositions immunogènes comprennent les hôtes cellulaires recombinants de l'invention.

L' invention vise par ailleurs des anticorps polyclonaux ou monoclonaux caractérisés en ce qu'ils reconnaissent une protéine recombinante ou polypeptide de fusion de l'invention, comprenant d'une part la séquence codée par le gène ou une partie du gène de la lipoprotéine et d'autre part, la séquence d'acides aminés codée par la séquence de nucléotides hétérologue.

Des anticorps monoclonaux particuliers selon l'invention sont en particulier caractérisés en ce qu'ils reconnaissent la séquence codée par le gène ou une partie du gène de la lipoprotéine et en ce qu'ils ne reconnaissent pas le polypeptide de fusion ci-dessus défini, comprenant d'une part la séquence codée par le gène ou une partie du gène de la lipoprotéine et d'autre part, la séquence d'acides aminés codée par la séquence de nucléotides hétérologue.

Un anticorps particulièrement intéressant est l'anticorps KF9 produit par l'hybridome KF9 déposé à l'ECACC (European Collection of Animal Cell Cultures Salisbury, Royaume-Uni)sous le n° 93073008 le 30 juillet 1993. Cet anticorps a l'avantage tout à fait intéressant de reconnaitre la lipoprotéine exprimée par le vecteur et de ne pas reconnaître cette lipoprotéine ou une partie de cette lipoprotéine lorsqu'elle est modifiée par un insert correspondant à une séquence de nucléotides hétérologue de taille supérieure à celle d'un multi-adaptateur. Ainsi cet anticorps peut par exemple être utilisé pour faire du criblage de l'expression des polypeptides de fusion de l'invention.

D'autres caractéristiques et avantages de l'invention apparaissent dans les exemples et les figures qui suivent.

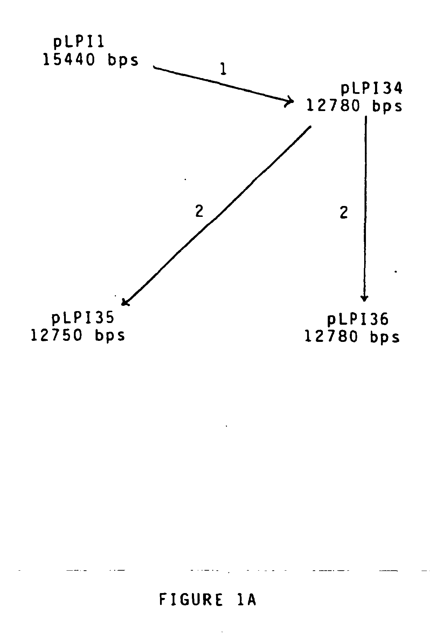

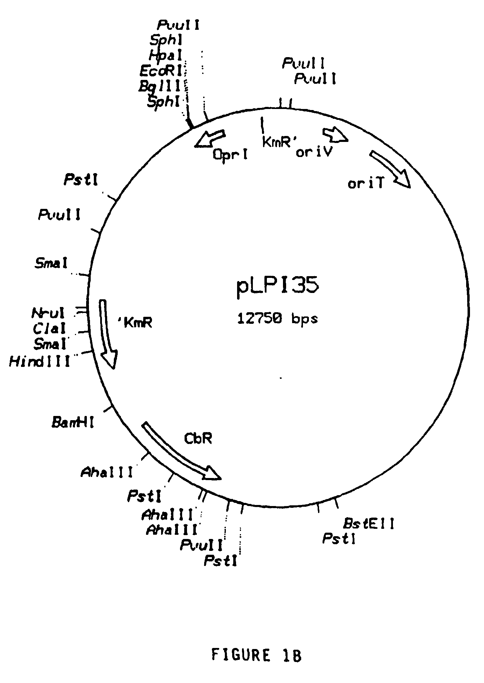

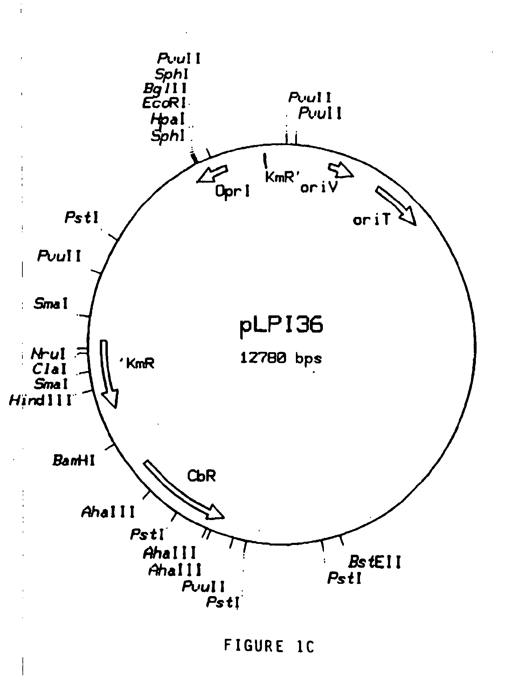

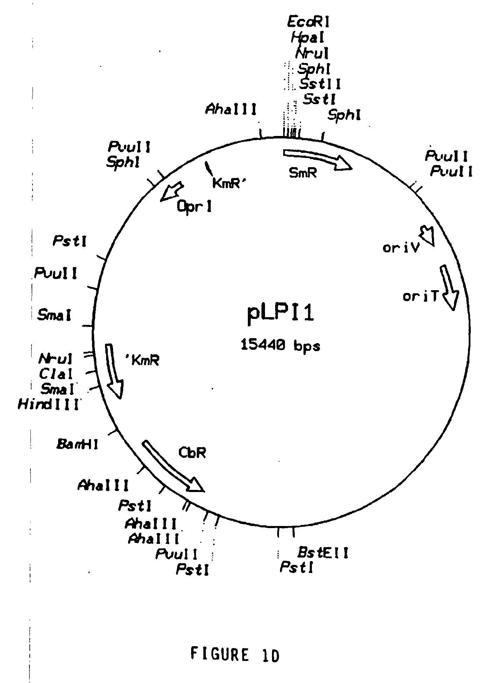

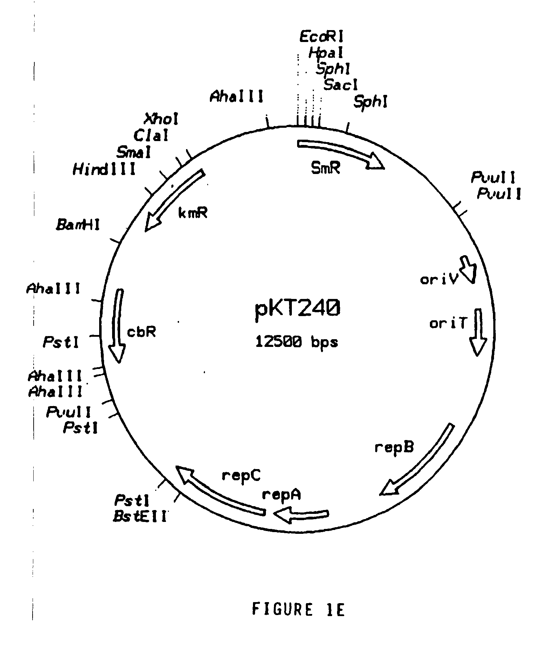

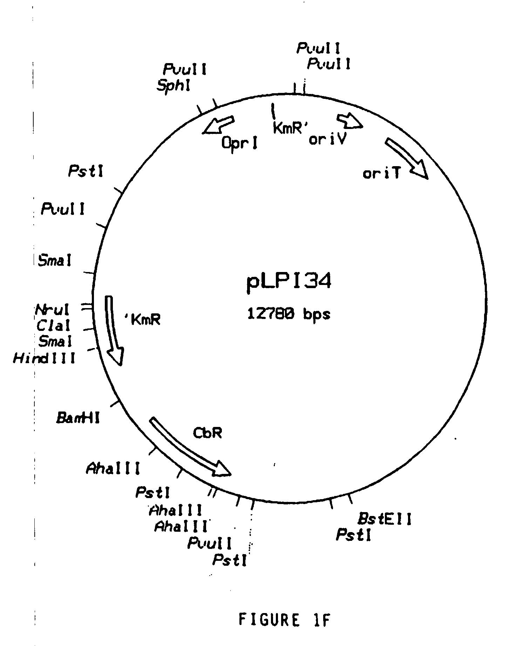

Figure 1 : Construction des vecteurs pLPI35 et pLPI36 pLPI35 et pLPI36 sont des vecteurs à faible nombre de copies permettant l'expression constitutive de la lipoprotéine dont la séquence codante a été modifiée par l'addition dans sa phase de lecture d'un polylinker.
   Le plasmide pLPI1 dérive du vecteur pKT240. Il contient un fragment SalI de la lipoprotéine de P. aeruginosa ayant une taille de 2,94 kb, clone dans le site XhoI du vecteur.
   L'étape 1 de la construction part du plasmide originel pLPI1 que l'on a coupé avec SstI et digéré avec Bal31 pour enlever progressivement de l'ADN dans les deux directions à partir du site SstI (selon les conditions décrites dans "Molecular Cloning - A laboratory manual second edition" - Sambrook et al (1989).
   Un clone qui produisait toujours la lipoprotéine de P.aeruginosa a été sélectionné par réaction positive avec un antisérum monoclonal anti-lipoprotéine a été sélectionné.
   La carte du plasmide obtenu, pLPI34 est représentée. pLPI34 a perdu 2 kb de DNA et les sites de restriction suivants: EcoRI, HpaI, NruI, SphI (2x), SstII, SstI. Le plasmide pLPI34 contient un site unique pour SphI vers la fin de la phase de lecture du gène de la lipoprotéine (OprI).
   L'étape 2 consiste en un clonage d'un polylinker avec extrémités cohésives SphI et contenant les sites HpaI (extrémités franches), EcoRI et BqlII. Les plasmides pLPI35 et pLPI36 contiennent le polylinker dans les deux orientations. Le polylinker a été conçu pour ne pas interrompre la phase de lecture, quelle que soit l'orientation de l'insert.
   Ces deux plasmides permettent l'insertion de fragments d'ADN coupés par les enzymes suivantes: BglII, BamHI, Sau3A et MboI (dans le site BglII), EcoRI et n'importe quel fragment à bouts droits (dans le site HpaI). Ils sont à faible nombre de copie (4 à 5) et sont conjugatifs, c'est à dire, qu'ils peut être transférés par conjugaison vers d'autres bactéries gram-négatives.
   L'expression de la lipoprotéine est consitutive dans ces vecteurs.
   Un anticorps monoclonal, KF9, reconnait toujours la lipoprotéine produite par pLPI36 mais ne réagit plus lorsqu'un fragment a été inséré dans un des sites de clonage cités plus haut.
   L'expression des protéines de fusion entre la lipoprotéine et l'insert est constitutive grâce au promoteur propre de la lipoprotéine.

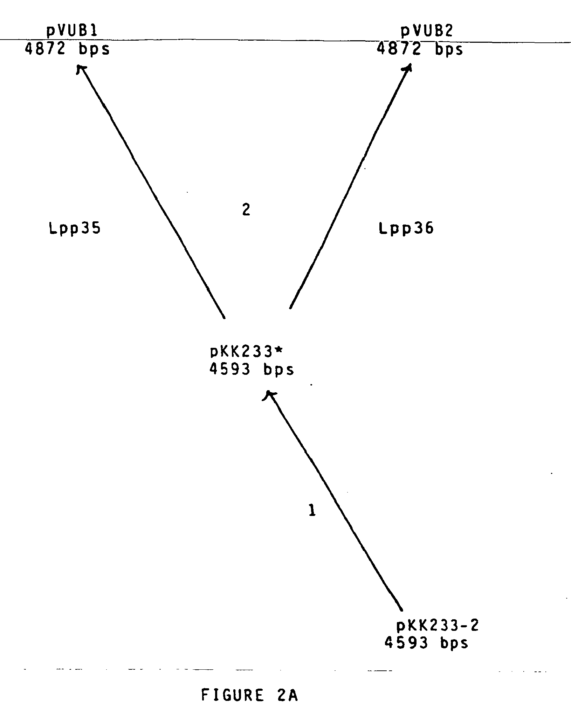

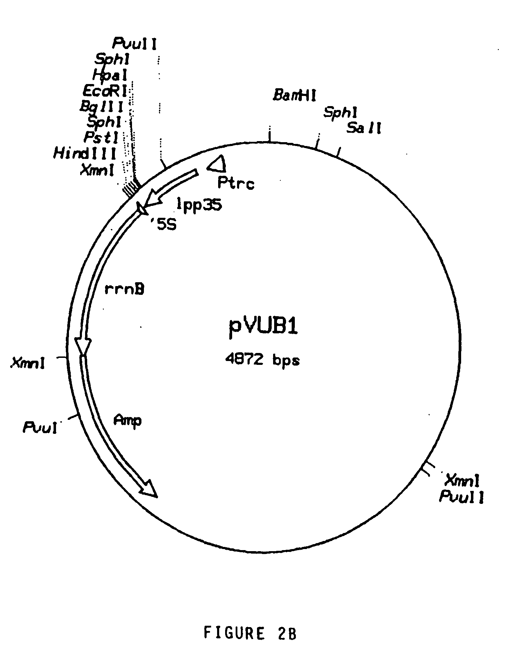

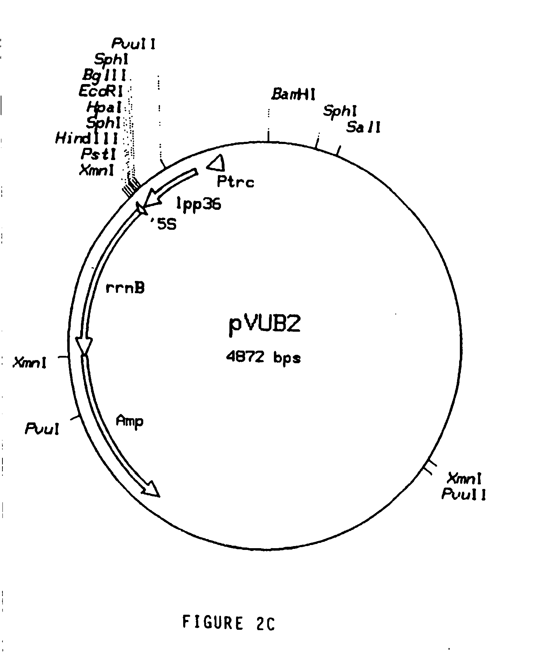

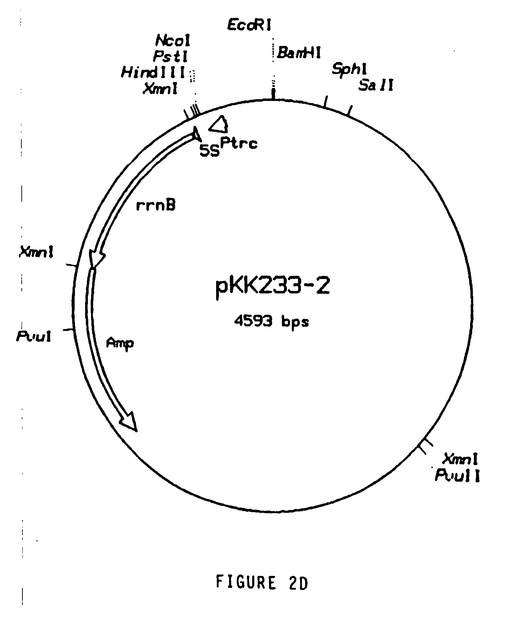

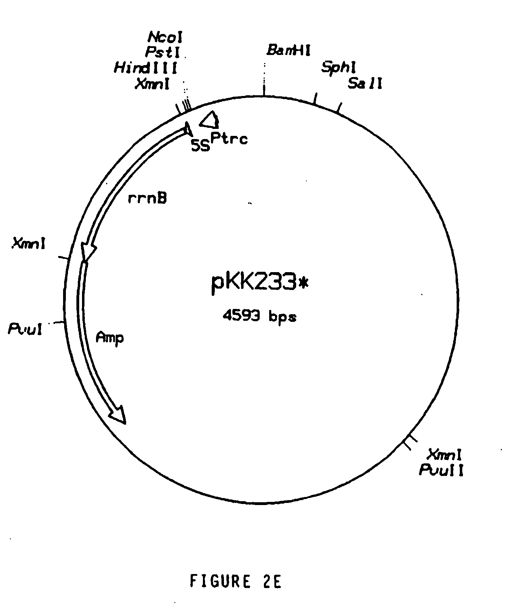

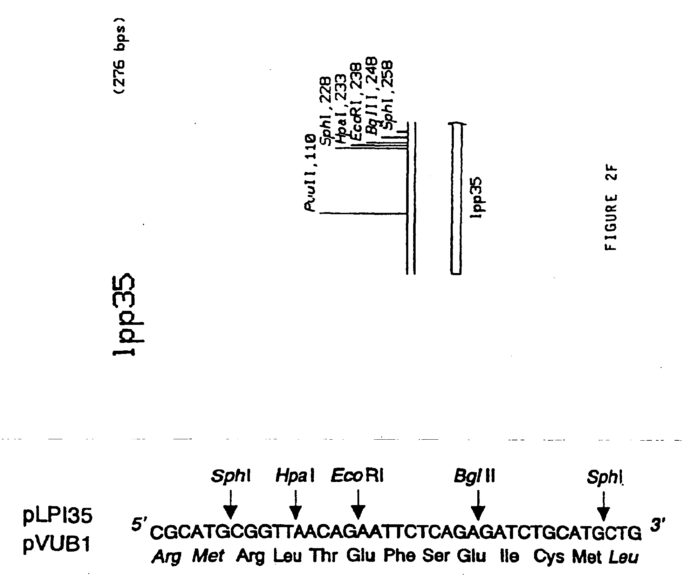

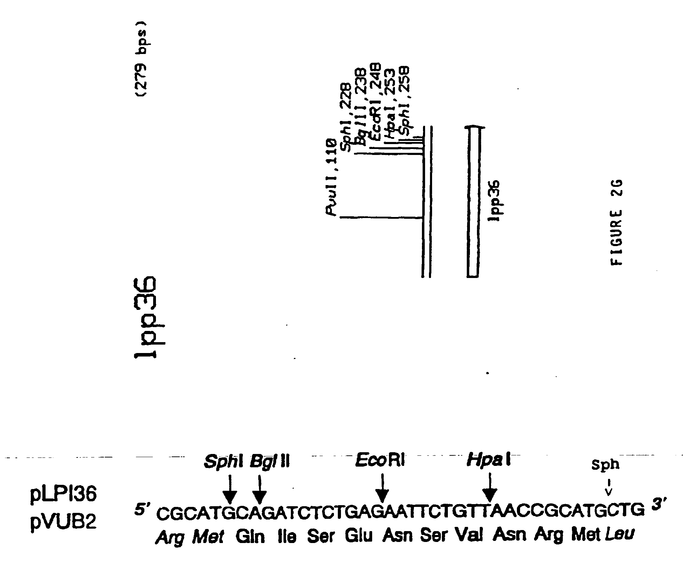

Figure 2 : Construction des vecteurs pVUB-1 et pVUB-2.
   Le vecteur d'expression pKK233-2 (Pharmacia) a été utilisé : son site EcoRI a été éliminé par restriction, puis un remplissage avec le fragment Klenow et une ligature ont été effectués.
   Le plasmide qui en résulte, pKK233* a ensuite été restreint par NcoI, traité par Klenow et ligaturé avec un fragment amplifié par PCR, correspondant à l'ORF entière du gène modifié de la lipoprotéine de pLPI35 ou de pLPI36 (lpp35 et lpp36, voir étape 2). Ceci a donné les deux vecteurs pVUB-1 et pVUB-2.
   Dans ces vecteurs multicopies l'expression de la lipoprotéine est contrôlée par l'IPTG. Afin d'éviter l'expression avant l'induction, il est préférable de réaliser des cultures en milieu B + Glucose ou en milieu M9 + Glucose.
   Les séquences des polylinkers utilisés pour construire les vecteurs pVUB1 et pVUB2 sont données dans les figures 2F et 2G.

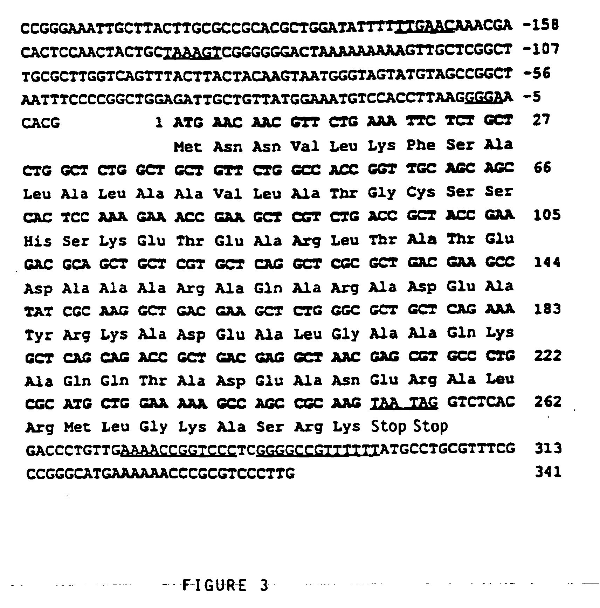

Figure 3: Séquence codante et séquence d'acides aminés correspondante de la lipoprotéine majeure de la membrane de Pseudomonas aeruginosa.
Figure 4 : (A) Clones E.coli JM 109 recombinants modifiés par le vecteur pVUB-1 contenant un fragment d'ADN E.stR1 d'un antigène de Emeria stiedae. Détection des clones positifs par hybridation radioactive avec la sonde E. stR1.
   (B) Marquage immunogold et microscopie électronique de E. coli JE5513 (lpp-) transformée avec le plasmide pLPI34 contenant le gène de la lipoprotéine de P. aeruginosa. Les cellules ont réagi avec l'AcM FA2.5 et des particules d'or (10 nm) couplées avec la Protéine A (grossissement 45 000 x).
Figure 5 : ADN extrait du clone E.coli JM 109 recombinant modifié par le vecteur pVUB-1 contenant le fragment E.stR1 d'un antigène de E.stiedae. Visualisation sur gel d'agarose 1-2% de l'ADN extrait.
   1 = marqueur λpst1
   2 = pVUB.1 (non digéré)
   3 = pVUB.1 (digéré par EcoRI)
   4 = pVUB.1/E.stR1 (non digéré)
   5 = pVUB.1/E.stR1 (digéré par EcoRI)
Figure 6 : (A) Analyse en western blot pour détecter la protéine de fusion par réaction avec un anticorps FA 2.5 dirigé contre la lipoprotéine.
   1 = marqueur
   2 = pVUB-1
   3 = pVUB-1/E.stR1
   (B) Séparation sur gel SDS-PAGE 17.5% des fractions membranaires et cytoplasmiques des extraits de protéine de pVUB.1 et pVUB. 1/E.stR1 
1 = Fraction membranaire
pVUB.1
2 = Fraction cytoplasmique
 
3 = Fraction membranaire
                                    pVUB.1/E.stR1
4 = Fraction cytoplasmique
   (C) Western Blot des fractions membranaires et cytoplasmiques des extraits de protéine de pVUB.1 et pVUB.1/E.stR1 : Criblage avec le monoclonal FA2.5 (anti-lipoprotéine) 
1 = Fraction membranaire ┐
                        | pVUB.35
2 = Fraction cytoplasmique┘
 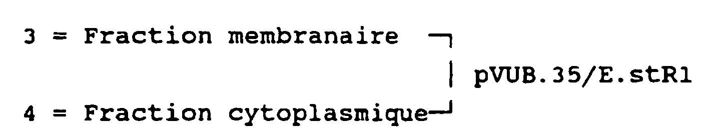
3 = Fraction membranaire
 pVUB.35/E.stR1
4 = Fraction cytoplasmique

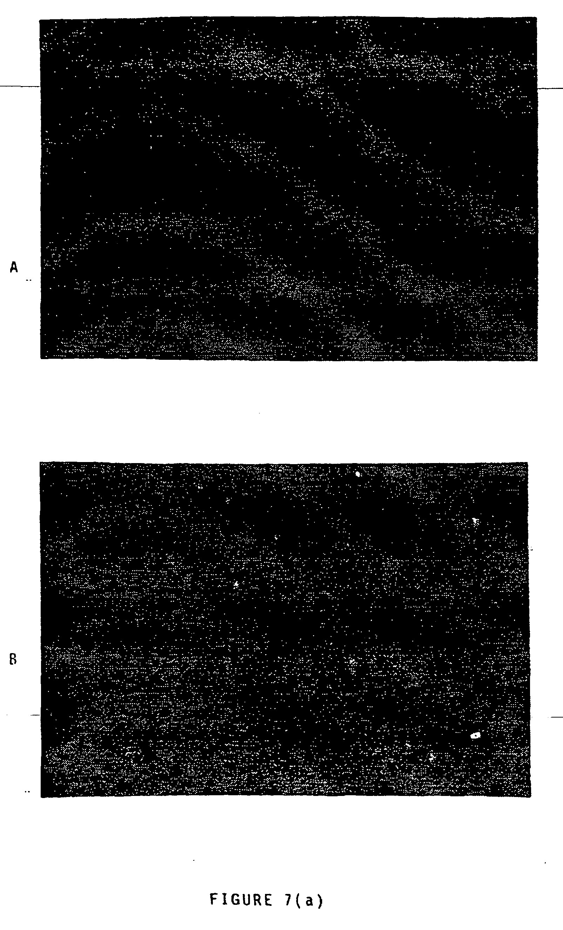

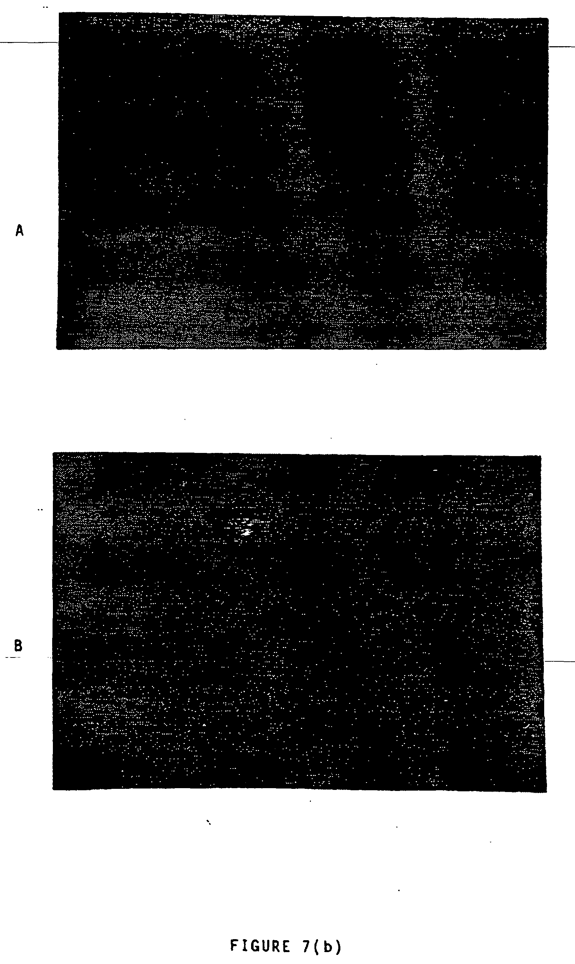

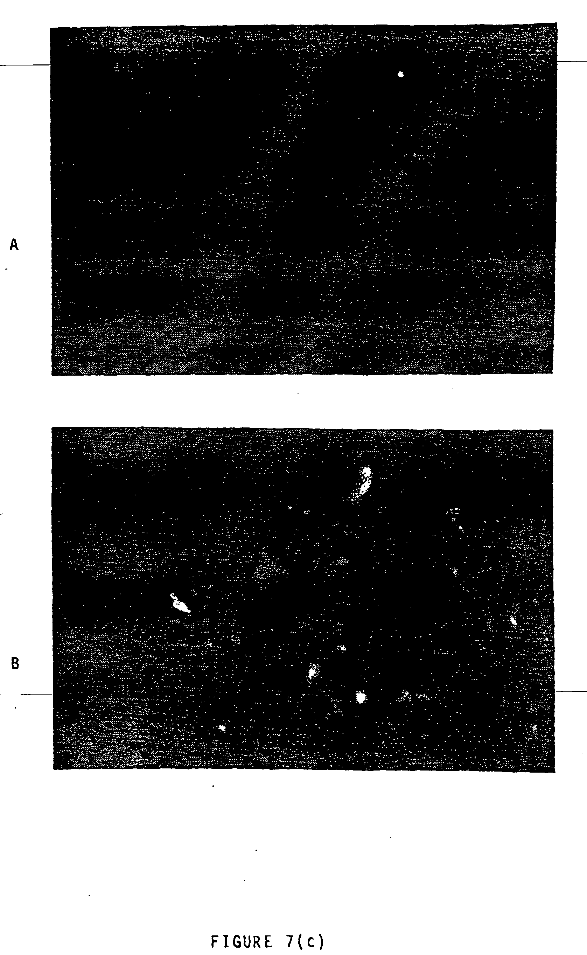

Figure 7 : (A) Immunofluorescence avec le sérum de lapin anti-S2b préalablement adsorbé avec E. coli JM109 transformée avec le plasmide pLPI35.
   (A) E. coli JM 109 avec pLPI35
   (B) Clone F3 : E. coli JM 109 transformée avec la fusion pLPI35-S2b. La séquence S2b contient des épitopes T et B.
      (B) Immunofluorescence avec le sérum de lapin anti-S2b préalablement adsorbé avec E. coli JM109 transformée avec le plasmide pVUB1.
   (A) E. coli JM 109 avec pVUB1, non induit
   (B) Clone F3 : E. coli JM 109 transformée avec la fusion pVUB1, induit par l'IPTG.
      (C) Immunofluorescence avec le sérum de lapin anti-S2b préalablement adsorbé avec E. coli JM109 transformée avec le plasmide pVUB1.
   (A) clone D10 = E. coli JM 109 avec la fusion pVUB1-S2b, non induit;
   (B) Clone D10 = E. coli JM 109 transformée avec la fusion pVUB1-S2b, induit avec l'IPTG. La séquence S2b contient des épitopes B et T.

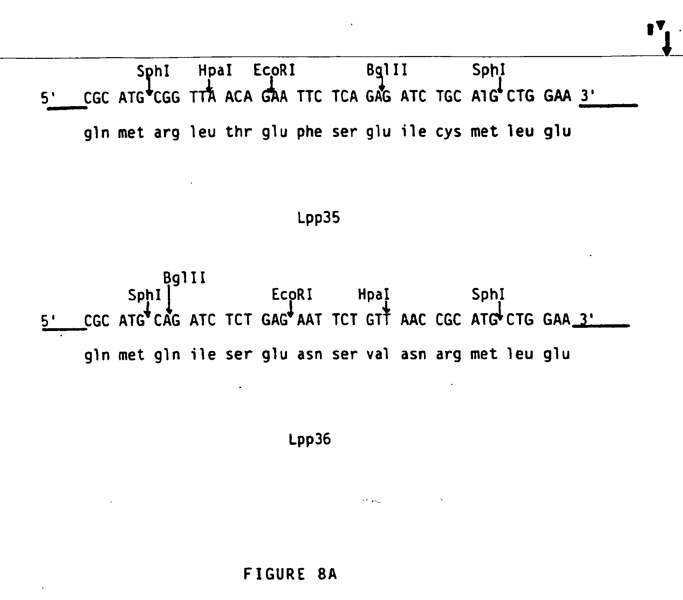

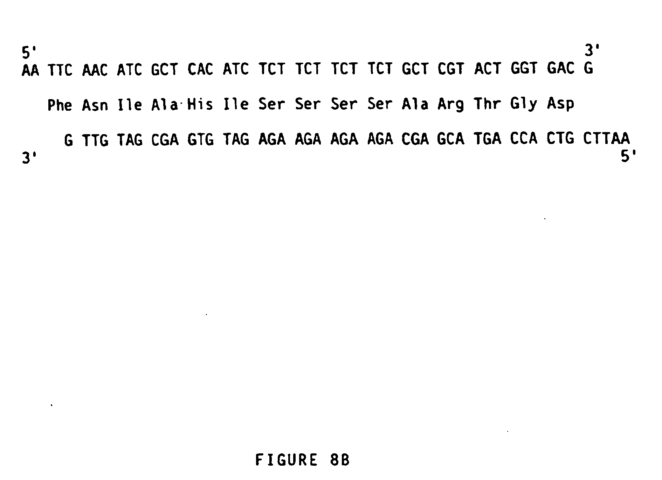

Figure 8 : - Insérat codant pour l'épitope T S2b de l'hépatite B cloné dans le site EcoRI de la lpp de P.aeruginosa.
   - Stratégie de clonage de l'épitope T S2b dans les vecteurs pVUB-1 et pVUB-2.

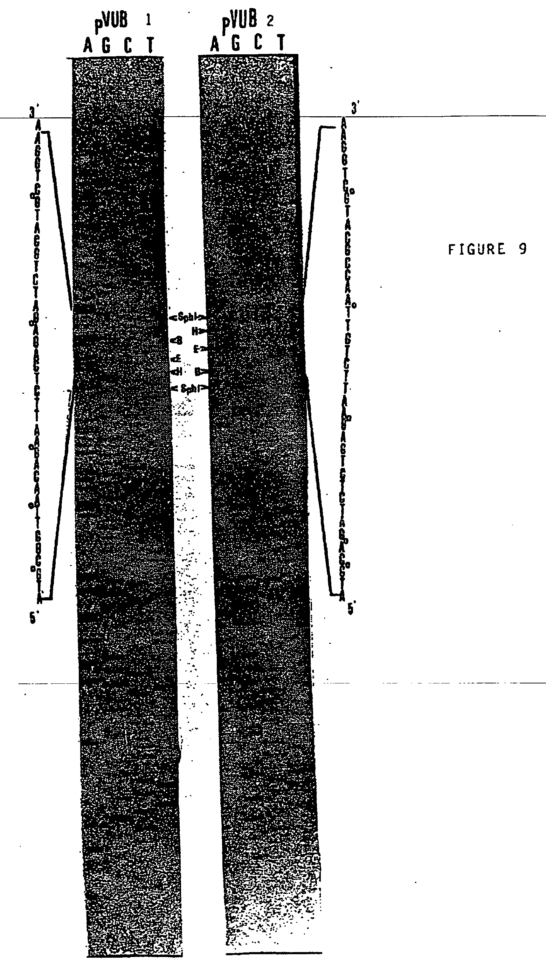

Figure 9 : Séquençage du polylinker en orientation contraire pour pVUB-1 et pVUB-2. Les sites de restriction du polylinker sont indiqués par les lettres H = HpaI, E = EcoRI et B = BglII.

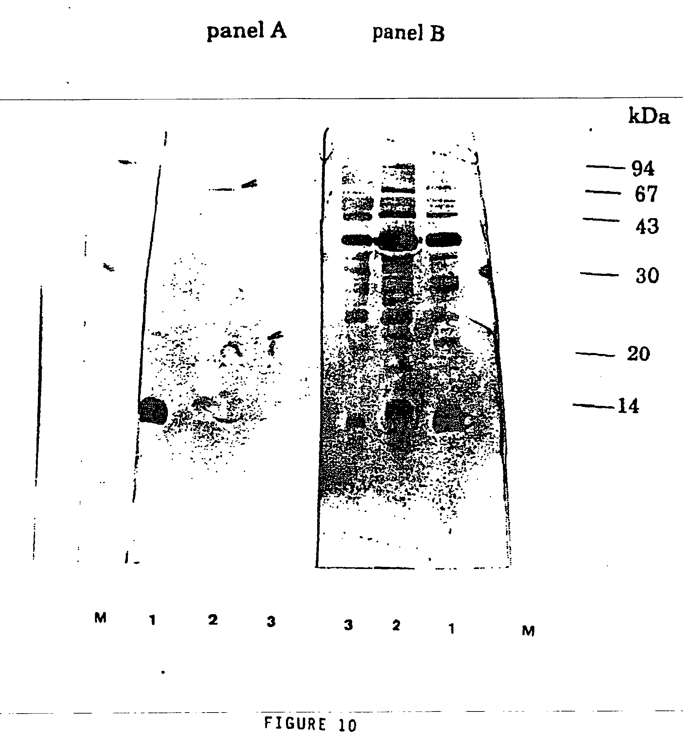

Figure 10 : Profil de la protéine et production de la lipoprotéine à partir de la préparation de membrane externe, par coloration au bleu de coomassie (a,b et c) et par western blot (d et e) en utilisant l'anticorps FA 2.5 anti-1pp.

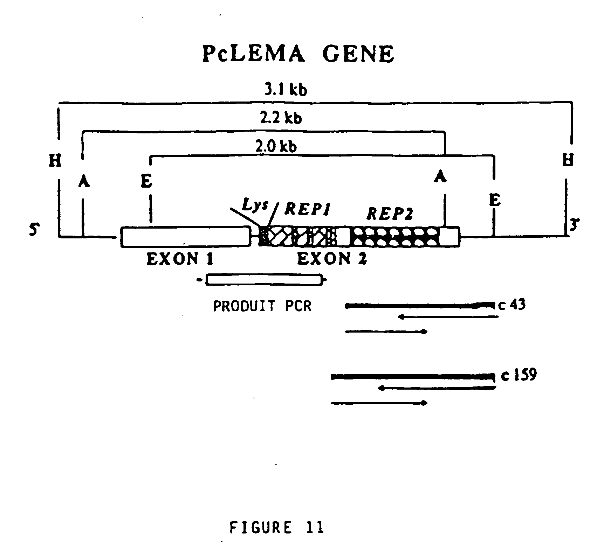

Figure 11 : Structure primaire du gène PcLEMA ; C43 et C159 sont les fragments d'ADNc reconnus par les anticorps monoclonaux; C159 est utilisé pour sous-cloner dans pVUB-1.

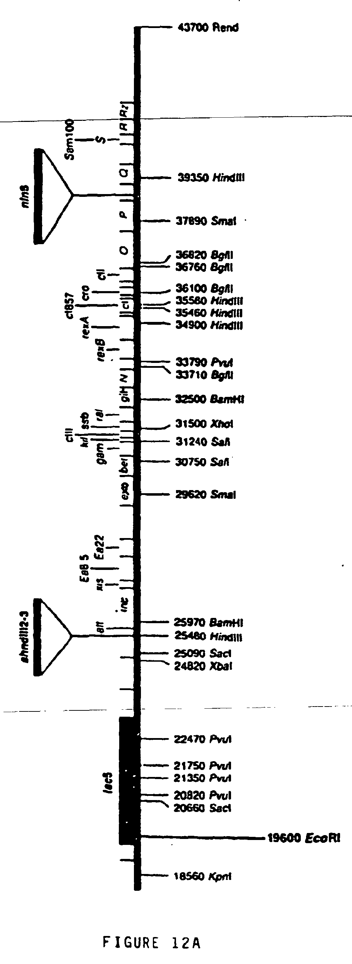

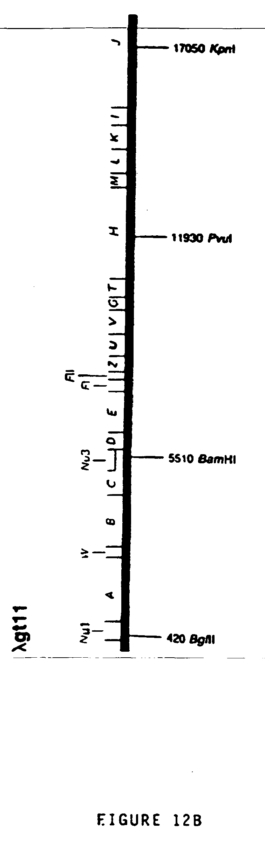

Figure 12 : Carte de restriction du vecteur lambda gt11.

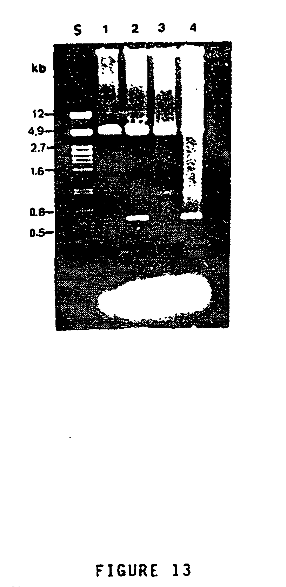

Figure 13 :Electrophorèse sur gel d'agarose 0,8%, de plasmides digérés par EcoRI: pVUB1 (ligne 1), pVUB1-650 bp PcLEMA (ligne 3), pVUB2 (ligne 4), pVUB2-650 bp PcLEMA (ligne 5) et lambda digéré par PstI, comme marqueur standard (ligne 1).

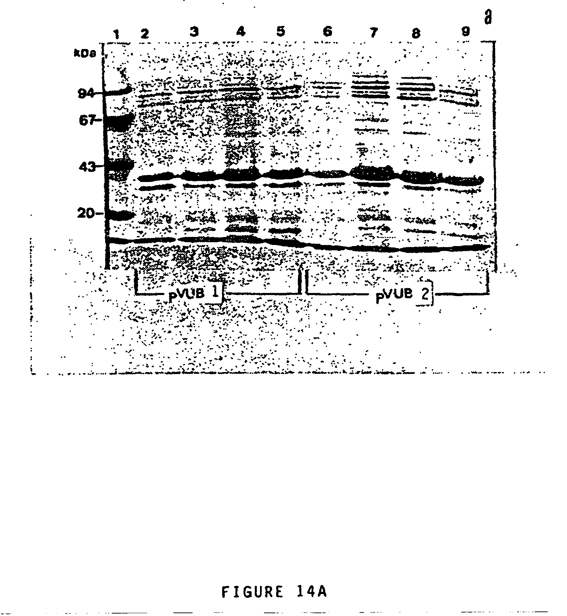

Figure 14 : Profil des protéines et production de Lpp à partir de préparations de membranes externes colorées au bleu de Coomassie (a, b, c) et en Western Blot, en utilisant l'anticorps monoclonal FA2.5 contre la Lpp. Préparation de membranes externes (a) de pVUB1 (lignes 2, 3, 4 et 5) et pVUB2 (lignes 6, 7, 8 et 9); absence d'induction, induction par IPTG pendant 1 h (lignes 3 et 7), 3 h (lignes 4 et 8), 6 h (lignes 5 et 9).
   Préparation de membranes externes de pVUB1 et pVUB2 (c); absence d'induction (ligne 2), induction par IPTG pendant 3 h avec addition de FeCl₃ 5 µM (ligne 3), 10 µM (ligne 4), 25 µM (ligne 5), 50 µM (ligne 6), 100 µM (ligne 7). La ligne 1 correspond au marqueur standard.

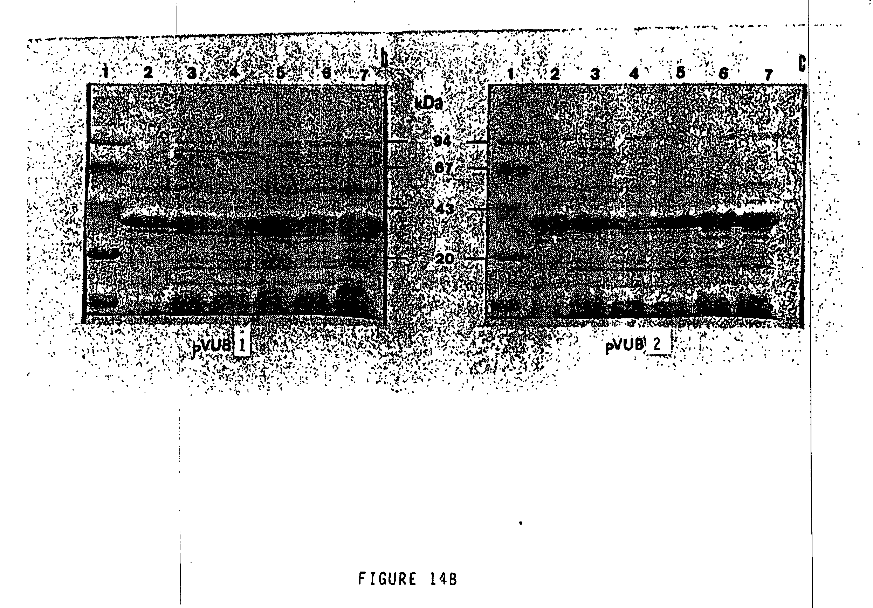

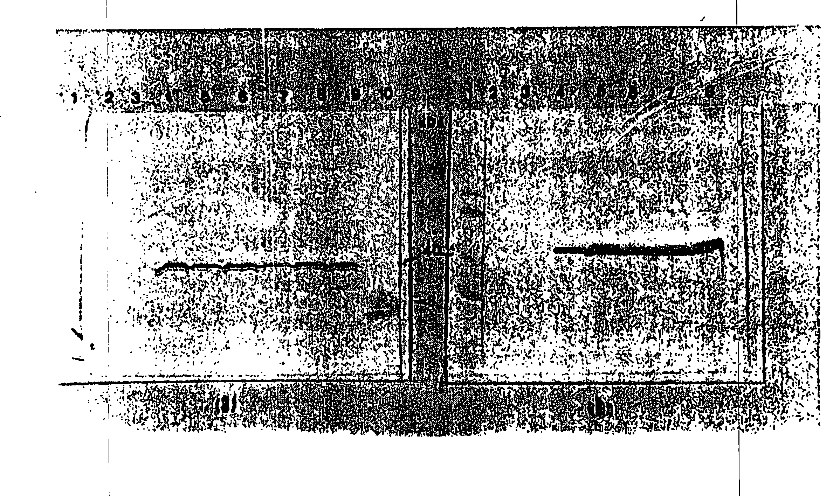

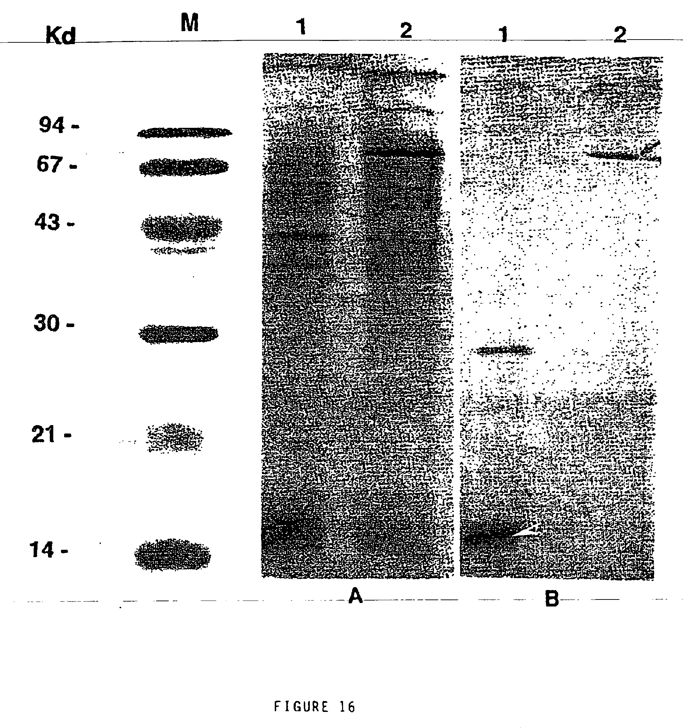

Figure 15 : Analyse Western blot de pLPI35 ou de pVUB1 fusionné avec de l'Ag PcLEMA détecté par l'AcM FA2.5 dirigé contre lpp (a) et l'AcM32 contre pcLEMA (b).
   L'extraction des protéines totales (a) en l'absence d'induction de pVUB1 (ligne 2), pLPI 35-650 bp PcLEMA (ligne 3), avec induction par l'IPTG de pVUB1 -650 bp PcLEMA pendant 30 min (ligne 4 et 7), pendant 3 h (lignes 5 et 8), 6h (ligne 6 et 9), induction à l' IPTG de pVUB1 pendant 3 h (ligne 10).
   L'extraction des protéines totales (b) en l'absence d'induction de pVUBI (ligne 2), avec induction à l'IPTG de pVUBI (ligne 3), pLPI 35-650 bp PcLEMA (ligne 4), avec induction à l'IPTG de pVUB1-650 bp PcLEMA pendant 30 min (ligne 5), 3h (lignes 6 et 7), 6h (ligne 7). La ligne 1 correspond au marqueur standard.
Figure 16 :
   A. SDS-PAGE des protéines totales de la membrane externe.
      1. pVUB2, induit pendant 5 heures
      2. pC7 gène gp63 de leishmania major cloné dans pVUB2) induit pendant 5 heures
   B. Western Blot des protéines totales de la membrane externe, testées avec un anticorps monoclonal FA2.5 anti-lipoprotéine.
      1. pVUB2 après induction pendant 5 heures (la flèche indique la lipoprotéine modifiée.
      2. pC7 après induction pendant 5 heures (la flèche indique la protéine de fusion).
Figure 17 :
   Production d'anticorps avec la protéine de coccidiose. La production d'anticorps a été testée après immunisation de 4 groupes de souris selon le protocole suivant :
      Groupe 1: 10 µg intrapéritonéal de β-gal-coccidiose en PBS
      Groupe 2: 10 *µ*g intrapéritonéal de β-gal-coccidiose + CFA
      Groupe 3: 10 *µ*g intrapéritonéal de lpp-coccidiose en PBS
      Groupe 4: 10 *µ*g intrapéritonéal de lpp-coccidiose + CFA
      Les résultats sont les suivants:
      - losanges: sérum préimmun
      - carrés: moyenne des 4 séra du groupe 1
      - cercles: moyenne des 4 séra du groupe 2
      - croix: moyenne des 4 séra du groupe 3
      - triangles: moyenne des 4 séra du groupe 4

### Exemple 1. Construction de vecteurs recombinants contenant la lipoprotéine de P. aeruginosa

### MATERIELS ET METHODES

### a. Construction des vecteurs comportant la lipoprotéine

Un vecteur pLPI obtenu par l'insertion d'une séquence de nucléotides SalI de 2,94 kb du gène OprI codant pour la lipoprotéine de P. aeruginosa, au site XhoI du vecteur pKT240 a été utilisé. pLPI1 (Cornelis P. et al. Molecular Microbiology (1989) 3(3) 421-428) contient donc la phase ouverte de lecture de la séquence codant pour la lipoprotéine de Pseudomonas aeruginosa.

Dans un premier temps, les sites de restriction non désirés, présents sur le plasmide comportant la phase ouverte de lecture de la lipoprotéine, ont été éliminés par digestion avec l'enzyme Ba131. S'agissant du plasmide pLPI1, une coupure par l'enzyme de restriction Sst1 a été réalisée préalablement à la digestion contrôlée par Ba131. La digestion conduit à l'obtention de pLPI34 qui se distingue de pLPI1 par la suppression d'un certain nombre de sites de restriction choisis. En particulier, les sites uniques EcoRI et HpaI ont été éliminés, ainsi que les deux sites SphI du vecteur.

On a ensuite défini des oligonucléotides comportant des sites de restriction qui n'étaient pas présents dans le plasmide de départ ni dans la phase ouverte de lecture de la lipoprotéine. Ces oligonucléotides multi-adaptateurs, encore appelés polylinkers, ont été synthétisés en phase solide en utilisant la technique au phosphoramidite, sous forme de deux oligonucléotides complémentaires, dans un synthétiseur Applied Biochemicals.

On a ensuite effectué l'hybridation des deux oligonucléotides et la ligature avec le vecteur coupé par l'enzyme SphI. On a ainsi obtenu deux vecteurs pLPI35 et pLPI36, selon l'orientation du polylinker inséré.

Le clonage du polylinker a ensuite été vérifié par analyse de restriction du fragment de la lipoprotéine préalablement amplifié par PCR.

La vérification de la production d'une lipoprotéine complète dans la membrane externe, après transformation de cellules données par le vecteur, a été réalisée par Western Blot.

Un autre vecteur, construit à partir du plasmide pKK233-2 (Aman E. et al, 1985, Gene 40:183-190) a été utilisé.

Dans un premier temps, le site EcoRI du vecteur d'expression pKK233-2 (Pharmacia) a été éliminé en digérant le vecteur par l'enzyme EcoRI puis en remplissant avec un fragment Klenow et en religant.

On a ainsi obtenu le vecteur pKK233^{*} dans lequel on a clone des fragments codant pour la lipoprotéine, modifiés par les polylinkers tels qu'obtenus à partir de pLPI35 et pLPI36, ces fragments ayant été préalablement amplifiés par PCR. Les amorces utilisées pour la réaction de PCR correspondaient au début et à la fin de la séquence ORF telle que décrite par De VosD et al, J. Gen. Microbiol. 139 (1993) 2215-2223. La seconde amorce utilisée ne contenait pas les deux codons STOP du gène oprI. Le clonage a été réalisé par ligature des extrémités franches dans le site NcoI rempli préalablement avec un fragment de Klenow, en aval du site de liaison aux ribosomes et du promoteur tac.

Le clonage du fragment a été vérifié par analyse de restriction des plasmides obtenus, pVUB-1 et pVUB-2.

Une vérification de la possibilité d'induire l'expression de la lipoprotéine a été réalisée après induction par l'IPTG.

### b. Croissance de ASFV, purification du virus et extraction de l'ADN

Le virus ASFV/L60 a été cultivé sur des macrophages de porc puis récupéré et concentré par centrifugation. Le virus a ensuite été purifié par centrifugation dans des gradients discontinus de sucrose. Une extraction de l'ADN du virus a été effectuée à l'aide de la protéinase K et de traitements au phénol.

Une restriction de l'ADN purifié par l'enzyme EcoRI a confirmé l'origine de l'ADN.

### c. Clonage de l'ADN de ASFV dans les vecteurs contenant la lipoprotéine

L'ADN-du-virus ASFV a été restreint avec Sau3A (|GATC) ou HaeIII (GG|CC). Les plasmides pLPI35 et pLPI36 ont également été restreints avec l'enzyme BglII (compatible avec les fragments Sau3A) ou avec HpaI (extrémités franches, pour ligature avec les fragments HaeIII).

On a ensuite effectué la ligature et la transformation des cellules hôtes.

Les 2000 clones obtenus ont été répartis sur des plaques de microtitration et répliqués.

Les clones ont été soumis à une hybridation de colonie avec des fragments Sau3A de ASFV marqués avec de la digoxigenine (Boehringer Mannheim) et les clones marqués ont été détectés à la lumière en utilisant le substrat AMPPD de la phosphatase.

Les clones positifs ont ensuite été testés avec deux anticorps monoclonaux contre la lipoprotéine, l'un réagissant avec les clones contenant un insert dans la lpp et l'autre ne réagissant pas avec la protéine de fusion (lpp modifiée par l'insert).

Les protéines de la membrane externe des clones les plus intéressants ont été préparées et analysées par SDS-PAGE et Western Blot avec un anticorps monoclonal reconnaissant la protéine de fusion.

Pour tester la réponse immunoproliférative, des membranes externes de E. coli n'exprimant pas la lpp (lpp-) et de E. coli modifiée par les plasmides pLPI35 et pLPI36 ainsi que des clones produisant la fusion 1pp-ASFV ont été préparées.

### d. Développement et caractérisation d'anticorps monoclonaux contre la lipoprotéine

Des souris FI ont été immunisées par injections des préparations de membranes externes de Pseudomonas aeruginosa dans l'adjuvant complet de Freund. Ces injections ont été réalisées dans les coussinets des pattes de souris. 9 jours plus tard, une fusion des lymphocytes prélevés chez les souris immunisées avec des cellules de myélome NSO a été réalisée. 10 à 14 jours suivant cette fusion, les hybridomes ont été testés par ELISA sur des plaques de microtitration sur lesquelles on avait étalé des membranes externes de P. aeruginosa.

Les clones positifs ont été recherchés par un test ELISA avec des membranes externes de E. coli (pKT240 lpp-) et de E. coli (pLPI1 lpp+).

Le clonage des hybridomes ayant une activité anti-lpp a été réalisé par dilution limite et expension. On a ensuite fait pousser les clones chez des souris. La détermination de la nature de l'épitope reconnu était effectuée en générant des délétions dans le gène de la lipoprotéine, par digestion par Bal 31.

Un test de détection de la réaction de différents anticorps monoclonaux produits par ces hybridomes, avec les différents dérivés de la lipoprotéine, y inclus les protéines de fusion, a été effectué. Les anticorps monoclonaux les plus intéressants ont ensuite été purifiés.

### RESULTATS

### a. Développement des vecteurs recombinants contenant la lipoprotéine de P. aeruginosa

Un premier ensemble de vecteurs de clonage a été développé avec le gène de la lipoprotéine (lpp) de P. aeruginosa, cloné dans un vecteur à faible nombre de copies, le plasmide pKT240. Après l'élimination de certains sites de restriction du vecteur par une digestion avec Bal 31, des polylinkers ont été définis, sous la forme d'oligonucléotides contenant des sites de restriction uniques pour le clonage en vue de l'insertion dans le même cadre de lecture que celui de la lipoprotéine et dans des conditions permettant de ne pas changer de façon significative la structure de la protéine.

Les polylinkers contenant des extensions SphI ont été introduits dans le site unique de restriction SphI présent près de l'extrémité de la phase ouverte de lecture de la lpp (ORF). Cette phase ouverte de lecture contenait 250 pb. Les différents vecteurs obtenus distingués en fonction de leurs sites de restriction, étaient les suivants :
pLPI35: HpaI, EcoRI, BgIII.
pLPI36: BglII, EcoRI, HpaI.
pLPI37: XhoI, SacI, XbaI, ClaI, BgIII, XmaI, SmaI.
pLPI38: SmaI, XmaI, BglII, claI, XbaI, SacI, XhoI.

Dans ces vecteurs, une lipoprotéine complète a pu être exprimée de façon constitutive sous le contrôle de son propre promoteur.

Cette lipoprotéine était, comme la lipoprotéine native, présente dans la membrane externe de cellules E.coli utilisées comme hôtes, comme le montrent l'électrophorèse SDS-PAGE et les tests Western Blot avec des anticorps monoclonaux anti-Lpp.

Pour rendre possible l'expression contrôlée de la protéine, un clone a été réalisé avec le vecteur d'expression pKK233-2. Tout d'abord, le site EcoRI a été éliminé de ce vecteur par digestion avec EcoRI remplissage avec un fragment Klenow et religature. Le fragment contenant la phase ouverte de lecture des plasmides pLPI35 et pLPI36 a été amplifié par PCR et sous-cloné dans le site NcoI préalablement rempli avec le fragment de Klenow, du plasmide modifié pKK233-2, en aval du promoteur tac. On a obtenu les vecteurs suivants :
pVUB-1: HpaI, EcoRI, BglII
pVUB-2: BglII, EcoRI, HpaI.

Le milieu LB permet l'induction de l'expression de la lpp car il contient de l'extrait de levure dont la composition est de nature à induire l'opéron lactose (en s'attachant comme l'IPTG au répresseur lacI). Aussi est-il préférable de travailler en milieu définie quelle que soit sa nature (CAA, M9, etc...). L'addition de glucose permet en plus d'exercer une répression catabolique. Il est d'ailleurs impossible d'obtenir des transformants de pVUB1 ou pVUB2 en milieu LB + Ampicilline (Ap), ces transformants étant seulement obtenus en milieu LB + glucose + Ap ou en milieu M9 + Ap ou CAA + Ap. En revanche, certains clones contenant un insert peuvent être obtenus par transformation directe en milieu LB (moindre toxicité).

Dans un milieu de culture LB, l'expression n'était pas réprimée en l'absence d'IPTG et conduisait à la mort cellulaire.

### b. Croissance et purification de l'ASFV-L60 et extraction de l'ADN du virus

Des cultures de macrophages infectés par le virus ont été utilisées pour collecter 10¹⁰ CPE₅₀ de ASFV-L60. Dans la mesure où le titre moyen en macrophages infectés dans la culture était de 10⁶ CPE₅₀/ml, il a fallu collecter l'équivalent de 10 litres de surnageant pour avoir suffisamment de virus. Les surnageants ont été collectés séparément et centrifugés pour obtenir le virus sous forme de culots.

Le virus a été purifié par centrifugation en présence de gradients discontinus de sucrose et soumis à un traitement à l'ADNase pour éliminer l'ADN de porc. La bande contenant le virus a été récupérée, recentrifugée et lavée.

L'ADN viral a été extrait par un traitement avec la protéinase K et des extractions au phénol. L'ADN de ASFV a été visualisé par électrophorèse sur gel d'agarose sous forme d'une unique bande d'environ 200 kb alors qu'on observait également un ADN de faible poids moléculaire (résistant à la RNase) sous la forme d'un nuage en bas du gel, correspondant probablement à l'ADN de porc dégradé avec l'ADNase.

Le profil de restriction avec EcoRI permettait de voir des bandes discrètes correspondant au profil prélablement observé pour l'ADN de ASFV/L60. Des similitudes ont également été observées avec les profils publiés pour l'ADN de ASFV bien que des différences aient pu être constatées.

### c. Clonage des fragments d'ASFV dans pLPI35 et pLPI36

L'ADN de ASFV a été restreint avec Sau3A et ligaturé aux deux vecteurs contenant la lpp, pLPI35 et pLPI36 préalablement coupés avec BgIII. Une autre partie de l'ADN de ASFV a été restreinte avec HaeIII et ligaturée par ses extrémités franches avec ces mêmes vecteurs coupés préalablement avec HpaI.

Après ligature et transformation, environ 2000 clones ont été répartis sur les plaques de microtitration et répliqués puis hybridés avec des fragments Sau3A de l'ASFV marqués avec la digoxygénine en présence d'un excès d'ADN de porc coupé non marqué.

45 clones positifs ont été détectés et 12 d'entre eux ont été sélectionnés. Les membranes externes ont été préparées et les protéines ont été séparées par SDS-PAGE. Les gels ont été soit colorés avec le bleu de Coomassie ou déposés sur de la nitrocellulose pour réaliser une immunodétection. Deux clones produisaient des grandes protéines de fusion, un produisait une protéine de 25 kDa et un autre une protéine de 70 kDa.

Une amplification par PCR de ces deux clones a révélé la présence d'un fragment inséré de 0,8 kb pour le clone produisant la protéine de 25 kDa et de 2,5 kb pour le clone produisant la grande protéine de fusion.

### d. Obtention et caractérisation des anticorps monoclonaux contre la lipoprotéine de P. aeruginosa

Plus de 20 hybridomes obtenus ont présenté une réaction spécifique avec la lipoprotéine de P. aeruginosa sans réaction croisée avec les lipoprotéines d'entérobactéries. Une analyse des épitopes a été réalisée sur des lipoprotéines tronquées progressivement par leur extrémité C-terminale (après digestion par SphI du plasmide pLPI34, puis digestion par Ba131, religature et transformation de cellules hôtes). Ceci a permis de révéler que certains anticorps détectaient un épitope N-terminal alors que d'autres réagissaient avec un épitope C-terminal.

L'un des anticorps monoclonaux, KF9, reconnaissait uniquement la lipoprotéine de pLPI36 modifiée par l'insertion de 10 acides aminés correspondant au polylinker, mais ne reconnaissait pas des dérivés du plasmide contenant des insertions plus longues.

Cet anticorps monoclonal présentait donc un intérêt pour l'application au criblage de clones pour détecter la présence de séquences insérées. Deux autres anticorps monoclonaux, QB2 et FA2.5 pouvaient détecter les protéines de fusion.

### DISCUSSION

On a donc démontré qu'il est possible d'utiliser le gène de la lipoprotéine de Pseudomonas aeruginosa comme antigène pour l'expression à la surface de cellules recombinantes, de séquences d'acides aminés choisies. L'introduction de polylinkers contenant différents sites de restriction peut également être réalisée au niveau de la séquence codant pour la 1pp, sans altérer le niveau d'expression ni la localisation de la protéine dans la membrane cellulaire externe.

Deux types de vecteurs ont été développés, une première catégorie permettant l'expression constitutive et une deuxième catégorie conduisant à l'expression contrôlée de la protéine de fusion obtenue à partir de la 1pp elle-même modifiée par insertion d'une séquence déterminée. Certains anticorps monoclonaux facilitaient le criblage des recombinants en ne présentant pas de réaction avec les clones ayant l'insertion de la séquence d'acides aminés choisie dans le gène de la lipoprotéine ou au contraire d'autres anticorps permettaient la détection de protéines de fusion.

### Exemple 2. Isolement et caractérisation d'un clone recombinant codant pour un antigène d'Eimeria Stiedae (Coccidiose)

### Introduction

La coccidiose est une maladie provoquée chez le poulet et chez le lapin, par un parasite protozoacie du gène Eimeria qui envahit généralement les cellules du tube digestif et des glandes associées.

Cette maladie entraine d'énormes conséquences économiques sur l'industrie des petits élevages.

La prophylaxie médicale consiste actuellement à utiliser systématiquement des produits coccidiostatiques dans les aliments des jeunes animaux, mais des problèmes secondaires importants sont apparus, dûs en particulier au développement des souches d'Eimeria résistantes aux médicaments.

Par conséquent une attention particulière a été dirigée vers la réalisation de nouvelles méthodes de lutte contre la coccidiose. Cette attention a été de plus en plus orientée sur l'immunoprophylaxie qui correspond à l'étude des vaccins biologiques pour le contrôle de ces parasites.

Les résultats suivants sont relatifs à l'espèce Eimeria stiedae, espèce pathogène chez le lapin.

Des 9 espèces qui infectent le tractus intestinal, Eimeria stiedae est la seule espèce qui se développe dans les voies biliaires.

Dans cette maladie hépatique, le foie est considérablement hypertrophié et sa surface est marquée de nodosités blanchâtres de grosseur variable.

### Méthodologie et Résultats

### 1. Parasite

Cette étude a consisté en premier lieu à infecter des lapins âgés de 5 semaines avec des oocystes sporulés d'E.stiedae. Trois semaines après l'infection, les oocystes sont isolés à partir du foie et des canaux biliaires.

### 2. Extraction de l'ADN d'E.stiedae

L'ADN d'E. stiedae a été extrait à partir des sporozoites.

Dans un premier temps, on a isolé des oocystes à partir du foie et des canaux biliaires des lapins infectés par E. stiedae (2.10⁸ oocystes/ml).

Les oocystes ont ensuite été lavés et placés dans. 2% de bichromate de potassium à la température ambiante pour une durée de 48 h, pour obtenir une sporulation.

Les oocystes sporulés ont ensuite été lavés jusqu'à la disparition du bichromate de potassium et broyées dans du PES :
Nacl:8g
KCl:0,2g
Na₂HPO₄H₂O:2,07g
KH₂PO₄:0,24g

Ce broyage se fait mécaniquement à l'aide d'un "Potter" pour permettre la libération des sporocystes.

Les sporocystes ont été incubés dans de la trypsine à 0,25% et de l'acide glycodéoxycholique à 0,1%, pour permettre la libération des sporozoites.

L'incubation a été réalisée dans un bain marie à 41°C pendant 1 h avec agitation. On a ensuite centrifugé pendant 10 minutes à 3000 rpm, et récupéré le culot contenant les sporozoites.

Les sporozoites isolés ont été lysés avec:
10 mM Tris pH8
25 mM EDTA
100 mM Nacl
1% SDS

Puis on a ajouté la protéinase K jusqu'à une concentration finale de 100 µg/ml.

Une incubation dans un bain marie à 37° C a ensuite été réalisée pendant 3 h suivie de l'addition successive de :
- 1 volume de phénol puis de la centrifugation à la température ambiante pendant 10 minutes (2x) ;
- 2 volumes d'ethanol 100%

Puis le précipité a été placé à -70°C pendant 30 minutes.

On a centrifugé pendant 15 minutes à 4°C, puis lavé le culot avec 70% d'ethanol, séché et dissout dans du TE pH8 :
10 mM Tris
1mM EDTA.

La concentration d'ADN obtenu a été déterminée par la densité optique à 600 nm.

### 3. Construction d'une banque génomique

Une banque d'ADN génomique a été construite dans le vecteur d'insertion et d'expression lambda gt11 (site EcoRI). Le sérum anti-sporozoïte de lapin a été utilisé pour cribler la banque et pour identifier les recombinants exprimant les antigènes d'E. stiedae.

Un clone positif a été isolé. L'extraction de l'ADN contenu dans le phage recombinant a permis d'identifier un insert de 1.2 kb (E. stR1).

### 4. Préparation de la protéine de fusion β-galactosidase

La préparation de l'antigène recombinant a été réalisée à partir des lysogènes lambda gt11.

La protéine de fusion a pu être purifiée directement par électrophorèse sur un gel SDS-PAGE.

La protéine obtenue (estimation sur gel) est de 138 kd dont 22 kd correspondent à une partie de la protéine du parasite.

### 5. Antisérum

Pour la production de l'antisérum spécifique de l'antigène recombinant E. stR1, des lapins SPF (dépourvus d'agent pathogène spécifique ou "specific pathogen free" de l'INRA de France, ont été immunisés à 3 semaines d'intervalle avec 3 doses de 100 *µ*g de protéine de fusion électroéluée.

La première dose a été émulsifiée avec l'adjuvant complet de Freund et les doses suivantes avec l'adjuvant incomplet de Freund. Le sérum a été obtenu 10 jours après la dernière immunisation. Ce sérum a été testé en immunofluorescence indirecte sur des sporozoites séchés sur des lames et en Western Blot sur un lysat brut de sporozoïte.

### - Immunofluorescence

Le test révèle une réaction positive du sérum au niveau de la surface du parasite d'E. stiedae ; mais sur des sporozoites vivants la réaction est négative. Ceci démontre que l'antigène n'est pas exposé à la surface du parasite.

### - Western Blot

Le test réalisé sur un lysat brut de sporozoites de trois différentes espèces :
- stiedae : coccidiose hépatique
- magna : " intestinale
- intestinalis : " intestinale
démontre que l'antisérum (SPF et E. stRl) reconnait une protéine prédominante de 40 kd.

Un sérum obtenu avant immunisation a été utilisé comme contrôle négatif.

Sur les extraits d'oocystes non sporulés d'E. stiedae, l'antiserum (SPF and E. stRl) réagit également avec une bande de 40 kd.

Ceci explique que l'antigène n'est pas synthétisé au stade sporozoïte du parasite vu puisqu'il est déjà présent dans les oocystes non sporulés.

### 6. Séquence du fragment d'ADN E. stR₁

L'insertion du fragment d'ADN E. stR1 a été utilisée pour transformer le plasmide pUC18 et le clone a été séquence, après avoir réalisé un clonage de délétion du fragment génomique avec l'enzyme Exonucléase III.

La séquence a été réalisée selon la méthode de Sanger.

La séquence de ce fragment de 1.2 kb a révélé une phase ouverte de lecture de 741 nucléotides qui débute au site EcoRI.

C'est une séquence qui ne présente pas de nucléotides répétitifs.

La protéine déduite à une longueur de 247 acides aminés avec un poids moléculaire de 27,5 kd.

La taille de la protéine de fusion produite par ce clone indique que 62% du total de l'insert de 1.2 kb code pour la protéine du parasite.

### 7. Clonage dans le vecteur pVUB-1

Afin d'amplifier la protéine intéressante, le fragment d'ADN génomique EstR1 a été clone dans le nouveau vecteur d'expression pVUB-1.

Ce vecteur a la propriété d'exprimer l'antigène comme protéine de fusion avec la lipoprotéine de Pseudomonas aeruginosa.

La phase de lecture du vecteur pVUB-1 est compatible avec le site EcoRI du phage lambda gt11, le clonage a donc été réalisé à l'intérieur du site unique EcoRI

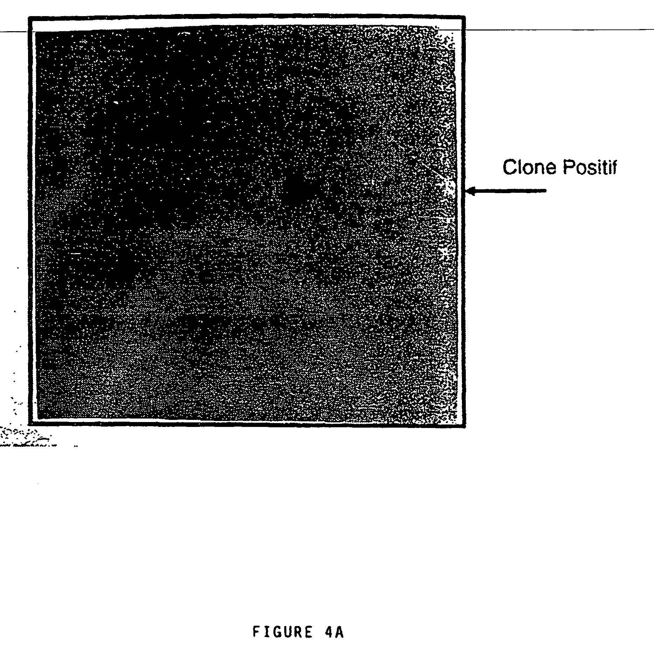

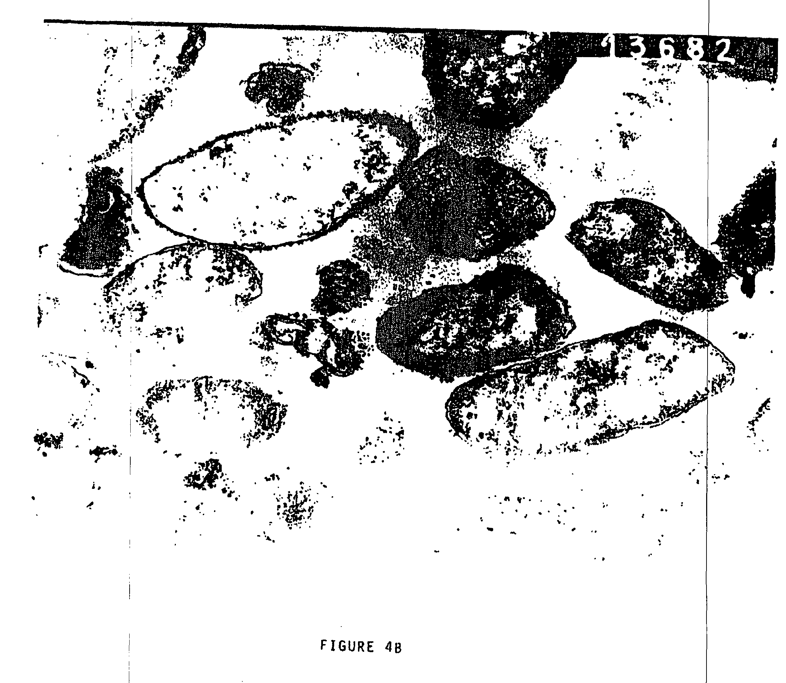

Après transformation de la bactérie E. coli souche JM109, les clones recombinants ont été sélectionnés par hybridisation et un clone positif a été isolé (figure 4).

L'analyse de ce clone a été réalisée par une extraction d'ADN plasmidique et par un Western Blot en immunodétection.

### - Extraction d'ADN plasmidique

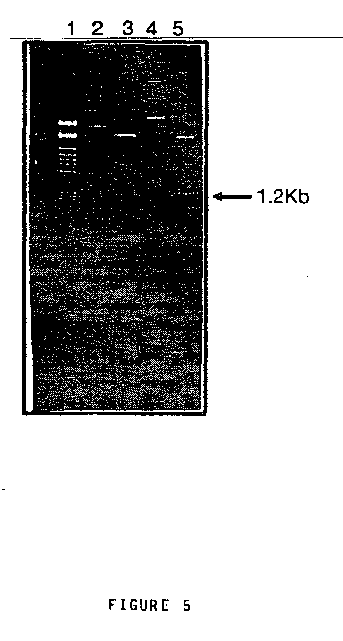

L'ADN, extrait du clone recombinant positif, a été digéré par l'enzyme de restriction EcoRI afin de visualiser l'insert de 1.2 kb (figure 5)

### - Western Blot

L'induction de l'expression de la protéine de fusion dans le plasmide pVUB-1 a été effectuée par l'addition dans le milieu de culture en présence d'IPTG.

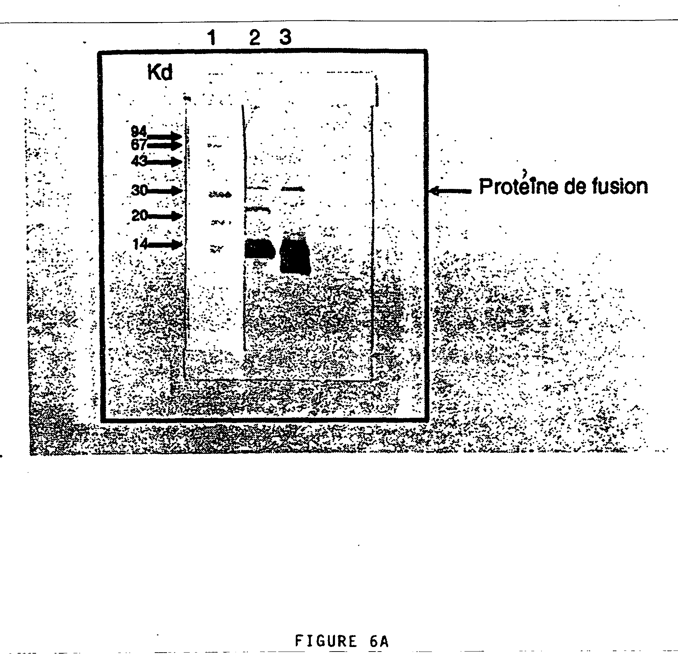

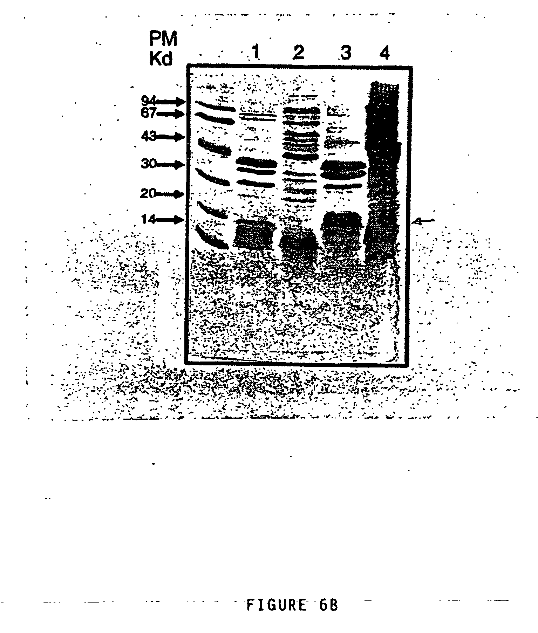

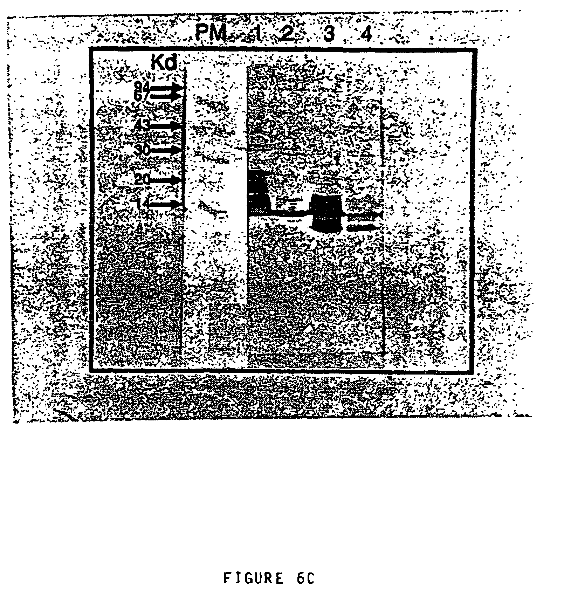

L'analyse en Western Blot a permis de vérifier la réaction de la protéine de fusion avec l'anticorps monoclonal FA2.5 dirigé contre la lipoprotéine. La protéine de fusion détectée a un poids moléculaire de 30-35 kd (figure 6).

### Conclusion

Les résultats des essais en immunodétection avec les antiserums SPF et E. stR1 sur les extraits bruts de sporozoïtes des différentes espèces testées (stiedae, magna, intestinalis et tenella :espèce la plus pathogène chez le poulet) ont montré l'existence de réactions croisées entre ces espèces du genre Eimeria.

Un avantage résultant de l'utilisation du vecteur pVUB-1 serait la possibilité de purifier la protéine en quantité suffisante pour permettre de tester l'activité immunologique et protectrice de l'antigène.

Pour réaliser ce test, la protéine recombinante, qui code pour un antigène de l'espèce étudiée Emeria stiedae, est amplifiée et utilisée pour des tests de vaccination.

Ces tests permettent de savoir si un tel "vaccin" pourrait contenir des antigènes communs aux différentes espèces et par là-même avoir un effet protecteur chez le lapin.

Le test concerne 3 espèces différentes :
- Eimeria stiedae
- " magna
- " intestinalis

Les paramètres évalués sont :
- le gain de poids de l'animal
- la conversion alimentaire
- le pourcentage de rejet d'oocystes par les excréments.

### Exemple 3.

Un insert de 48 pb, codant pour l'épitope T S2b de l'hépatite B et pour un épitope B, a été cloné dans le site EcoRI de la lipoprotéine de Pseudomonas aeruginosa. L'insert était formé par hybridation de 2 oligonucléotides complémentaires, générant des prolongements simple brin à chaque extrémité, compatibles avec les extrémités cohésives générées après la restriction avec l'enzyme EcoRI. L'insert était défini de façon telle qu'il permettait de maintenir la phase ouverte de lecture (ORF) de la lipoprotéine ainsi que l'insert lui-même (Figure 8).

Les vecteurs utilisés étaient : pVUB1 (environ 5 kb, grand nombre de copies contenant un promoteur de type tac (par exemple le promoteur trc) inductible par l'IPTG ou le lactose et ses dérivés, résistant à l'ampicilline, et pLPI35 (13,5 kb, faible nombre de copies, expression constitutive de la lipoprotéine, résistance à l'ampicilline).

La transformation de cellules de E. coli JM109 a conduit à l'obtention de plus de 250 transformants sélectionnés sur la base de leurs propriétés de résistance à l'ampicilline. Ces transformants ont été criblés pour rechercher la présence de l'épitope par empreinte de colonies en utilisant un antisérum polyclonal dirigé contre l'épitope S2b dans des lapins. Tous les anticorps qui ont présenté une réaction croisée ont été éliminés par adsorption contre un lysat cellulaire de E. coli JM109 transformé avec pVUB1.

La réaction secondaire a été effectuée avec la protéine A couplée à la peroxidase de Raifort, en utilisant un milieu 4-chloronaphtol (fraîchement préparé) et le péroxidase d'hydrogène de BIORAD comme substrat coloré.

Parmi les 277 colonies testées, 6 ont réagi clairement positivement.

La détection a également été réalisée par immunofluorescence.

### Exemple 4 : Construction d'un vecteur contenant la lipoprotéine de Pseudomonas aeruginosa dans des conditions telles que son expression peut être contrôlée dans E.coli.

### 1. construction de la lipoprotéine de P. aeruginosa dans le vecteur d'expression contrôlée pkk233-2, dans E. coli.

Un gène codant pour la lipoprotéine de P. aeruginosa a été clone dans pKT240 et séquencé (Cornelis P. et al, 1989). En conséquence, des dérivés de ce clone ont été construits et insérés dans un polylinker près de l'extrémité C-terminale du gène de structure lpp (c'est à dire dans pLPI35 et pLPI36). pLPI35 et pLPI36 contiennent un oligonucléotide de 30 mer avec un unique site de restriction pour HpaI, EcoRI et BglII en orientation opposée, ces sites ayant été insérés dans le site SphI. Ce gène contient son propre promoteur, ce qui conduit à une surproduction de lpp dans E. coli qui cause une réduction significative du taux de croissance de la bactérie.

Pour surmonter ce problème, on a considéré l'expression sous contrôle du promoteur fort trc de pKK233-2. Comme le plasmide contient le promoteur trc, le site de liaison au ribosome de lac-Z, et un codon d'initiation ATG, alors l'expression du gène lpp inséré sous le contrôle de ce promoteur pouvait être contrôlée par induction en utilisant de l'IPTG.

Comme un site EcoRI unique avait été introduit dans le polylinker du gène lpp, le site EcoRI du vecteur pkk233-2 a dû être éliminé. L'élimination de ce site a été suivie d'une ligature des extrémités franches de façon telle que le nouveau plasmide dérivé pKKD ne contenait plus le site EcoRI. A partir du plasmide pKKD, la construction du gène de structure lpp contenant le polylinker a été ligaturé au site NcoI dont les extrémités avaient été rendues franches avec le fragment de Klenow. Les clones recombinants pVUB1 et pVUB2 ont été obtenus comme dérivés de pLPI35 et pLPI36.

La stratégie de clonage est montrée à la figure 8 et le séquençage de pVUB1 et pVUB2 y compris le polylinker est à la figure 9.

### Exemple 5 : Expression de lpp dans E. coli

Des anticorps monoclonaux contre la lpp de P. aeruginosa ont été produits. Leur caractérisation a montré que certains d'entre eux reconnaissent des épitopes de la partie N-terminale (anticorps FA2.5, pE4.1) et d'autres reconnaissent un épitope proche du polylinker de pVUB2 (anticorps KF9). L'anticorps KF9 reconnait la Lpp de pLPI34 et non spécifiquement celle de pLPI36 ou pVU2. Des clones recombinants contenant le gène de la lpp ont été criblés par immunodétection de colonies. L'expression contrôlée des vecteurs pVUB1 et pVUB2 a été réalisée soit dans un milieu M9 plus acide casamino ou dans un milieu LB plus 1% de glucose contenant 100 *µ*g/ml ampicilline. L'induction a été réalisée avec 1mM d'IPTG (concentration finale) soit dans le milieu M9 contenant l'acide casamino ou dans le milieu LB contenant 100 *µ*g/ml d'ampicilline. Une incubation a été réalisée à 37°c pendant 3 à 6 heures. Pour déterminer le profil de la protéine et localiser le produit lpp, une préparation de membrane externe a été effectuée puis analysée soit en Western Blot soit en coloration avec le Bleu de Comassie. La figure 10 montre le profil de la protéine et la production de lpp par détection avec l'anticorps FA2.5. Le résultat montre une forte production de lpp à la fois sous forme libre et sous forme liée.

Pour augmenter la production de lpp, du fer (III) a été ajouté dans le milieu de culture. La surproduction de lpp a augmenté significativement dans 50 et 100 *µ*m de fer (III) supplémentaire (figure 10).

### Exemple 6 : Expression d'une protéine de fusion contenant la lpp et un antigène de la malaria.

Un antigène de la malaria spécifique du stade tardif a été utilisé. Il est obtenu de Plasmodium chabaudi qui est responsable de la Malaria des rongeurs permettant de définir un modèle pour la Malaria, chez la souris (Deleersnijder W. et al, Mol. Biochem. Parasitol. 43 (1990) 231-244). Cet antigène est associé avec la membrane des érythrocytes infectés et est appelé PcLEMA. La structure primaire du gène est donnée à la figure 11. Des clones d'ADNc contenant la partie C-terminale ont été criblés dans une banque d'expression d'ADNc construite dans lambda gt11 en utilisant un anticorps monoclonal contre cet antigène. Les inserts contiennent 650 et 600 pb respectivement, en phase avec le gène de la β-galactosidase, au site EcoRI.

Le vecteur pVUB1 contient un site EcoRI dans le polylinker, qui est compatible avec un site EcoRI dans le polylinker du gène de la β galactosidase du vecteur lambda gt11 (Figure 12). C'est pourquoi l'insert de 650 pb a été excisé par l'enzyme EcoRI et directement sous clone soit dans pLPI35, ou dans pVUB1 au site EcoRI. Après le criblage immunologique des colonies, les clones recombinants ont été analysés par extraction du plasmide (Figure 13) et Western Blot (Figure 14).

Le résultat montre que le produit de fusion de la lpp avec l'antigène de la malaria dans le vecteur d'expression contrôlée pVUB1, a un rendement de production supérieur à celui obtenu en exprimant cette même protéine de fusion à l'aide du vecteur pLPI35. Le poids moléculaire du produit de fusion est environ 30 kDa (Pm calculé) (8 kDa pour la lpp et 22 kDa pour l'insert de 650 pb), mais la migration en électrophorèse SDS-PAGE est détectée à environ 40 kDa. Ceci correspondrait à une zone extensive de répétitions en tandem et au caractère hydrophile prononcé de l'antigène de la malaria:

### Exemple 7 : Clonage et expression de la glycoprotéine de surface gp63 de Leishmania dans le vecteur pVUB2 contenant la lipoprotéine.

### 1) Clonage du gène de la qp63

Le gène entier de la gp63 de Leishmania major, présent sur un fragment EcoRI de 2,2 kb dans le vecteur Blue Script PBS (stratagène) a été décrit par Button et Mac Master (1988), J. Exp. Med., 176,724-729.

Le vecteur PBS a été digéré par EcoRI et le fragment de 2,2 kb a été purifié sur un gel d'agarose 1% en utilisant le kit Geneclean (BIO 101 Inc., La Jolla, Californie). Le fragment a ensuite été ligaturé au site EcoRI de pVUB2, qui était en phase avec la séquence de gp63. Le vecteur pVUB2 avait déjà été préparé par digestion avec EcoRI, suivi d'un traitement avec la Phosphatase Intestine de Veau (CIP), de façon à éviter l'autoligature du plasmide. pVUB2 avait finalement été purifié en utilisant un gel d'agarose 1% à l'aide du kit Geneclean. Après transformation de E. coli JM109, les transformants ont été analysés par analyse de restriction et les clones contenant le fragment de 2,2 kb ont été sélectionnés en tant que recombinants. L'orientation du fragment a également été analysé par analyse de restriction.

### 2) Expression du recombinant gp63.

L'expression de la protéine de fusion par les clones recombinants a été analyséee en électrophorèse SDS-PAGE. Une colonie de vecteur pVUB2 exprimant la gp63 a été cultivée pendant une nuit dans un milieu M9-CAA à 37°C. La culture a été diluée et cultivée à 37°C sous agitation constante jusqu'à obtenir une densité optique de 0,3 avant d'ajouter de l'IPTG (isopropyl B-D-thiogalactopyranoside) à 1 mM. Après 5 à 6 heures supplémentaires à 37°C, les bactéries ont été récupérées par centrifugation et la protéine recombinante de..fusion a été analysée par électrophorèse SDS-PAGE.

### 3) Purification de la protéine de fusion comportant la gp63

La protéine recombinante gp63 a été exprimée dans un litre de culture, selon la technique décrite précédemment. Les protéines contenues dans la membrane externe ont été préparées en faisant appel à une lysozyme puis à un traitement aux ultra-sons et ont été dissoutes dans 25 mM de Tris-HCl à pH 8. Les protéines ont ensuite été solubilisées dans un échantillon de tampon SDS et séparées par électrophorèse SDS-page sur des gels à 6%. Les gels ont été colorés dans du bleu de Coomassie dans 0.06% dans l'eau et décolorés avec de l'eau. La bande correspondant à la protéine de fusion a été découpée à partir du gel et purifiée par électroélution.

### 4) Production d'anticorps contre la gp63 recombinante

70 *µ*g de protéines de fusion gp63 purifiées par électroélution ont été injectés par voie souscutanée à deux lapins en l'absence d'adjuvant complet de Freund. Les lapins ont subi un rappel deux fois avec 60 *µ*g de gp63 recombinante purifiée à 4 semaines d'intervalle. Des sera normaux et immuns ont été obtenus à partir des lapins immunisés avant et une semaine après la première et la troisième immunisation respectivement.

### Exemple 8 : Production d'anticorps

### 1) avec la protéine de la coccidiose (Emeria stiedae):

La protéine de fusion décrite dans l'exemple 2 a été découpée d'un gel SDS-PAGE à 17.5% après migration des protéines de la membrane externe d'un clone E. coli contenant le plasmide recombinant décrit dans le texte et après induction de la culture par l'IPTG. Le gel a été coloré dans du bleu de coomassie (0.06%) dilué dans l'eau. La protéine de fusion a ensuite été électroéluée d'après les recommandations du fabricant (Bio-Rad). En parallèle le même antigène, mais fusionné à la β-galactosidase (clone lambda GT11) a été préparé et électroélué (à partir d'un extrait brut du clone E. coli).

Quatre groupes de 4 souris Balb/C ont été préparés pour les expériences d'immunisation:
Groupe 1: 10 µg intrapéritonéal de β-gal-coccidiose en PBS
Groupe 2: 10 µg intrapéritonéal de β-gal-coccidiose + CFA
Groupe 3: 10 µg intrapéritonéal de lpp-coccidiose en PBS
Groupe 4: 10 µg intrapéritonéal de lpp-coccidiose + CFA

Le volume injecté était chaque fois de 150 µl.

Quatre semaines après la première immunisation une deuxième immunisation a été effectuée, cette fois sans CFA, avec la même quantité de protéine ; une troisième immunisation a été effectuée 4 semaines après la deuxième toujours sans CFA. Dix jours après cette dernière immunisation, le sérum des souris a été prélevé et la présence d'anticorps testée en ELISA sur plaques microtitres (NUNC) contenant de l'extrait brut d'Emeiria stiedae provenant d'oocytes sporulés (10 µg/puit)**.**

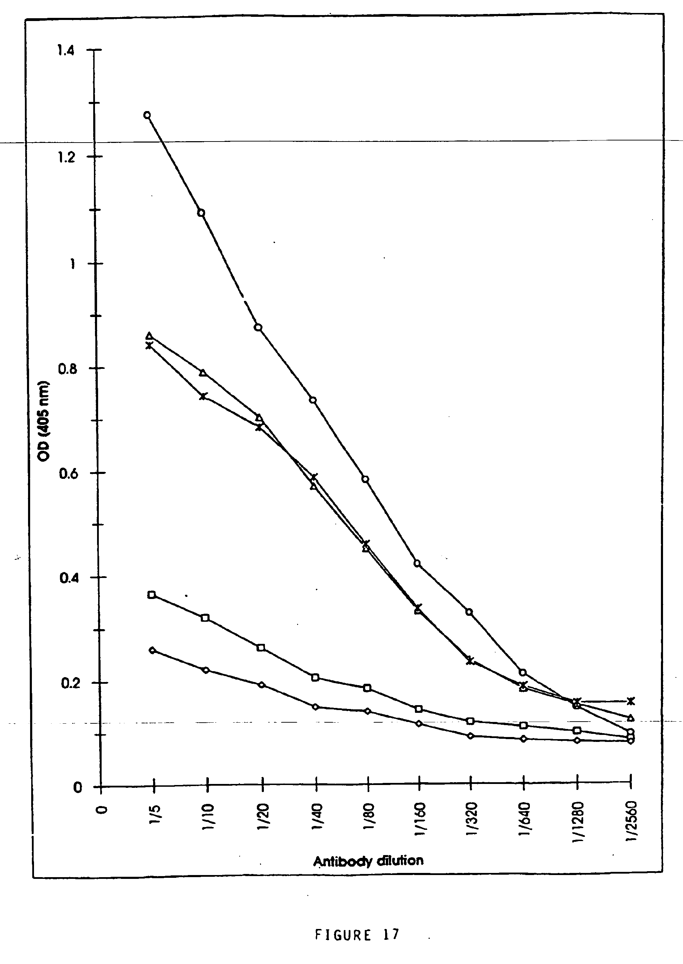

Après saturation des plaques à la BSA, les sera sont ajoutés à différentes dilutions, incubés deux heures et, après deux lavages au PBS-Tween, l'anticorps secondaire (chèvre anti-souris-phosphatase) est ajouté et l'incubation est poursuivie pendant 1 heure. Après 5 lavages, le substrat de la phosphatase est ajouté (p-nitrophényl phosphate) et la plaque est lue après une demi-heure d'incubation à température ambiante. Les résultats sont donnés dans la figure 17:
- losanges: sérum préimmun
- carrés: moyenne des 4 séra du groupe 1
- cercles: moyenne des 4 séra du groupe 2
- croix: moyenne des 4 séra du groupe 3
- triangles: moyenne des 4 séra du groupe 4

### 2) avec un antigène de la malaria de Plasmodium chabaudi:

3 La protéine de fusion produite à partir du clone pVUB et décrite dans l'exemple b a été similairement éluée (voir 1) et injectée avec ou sans CFA (20 µg de protéine) dans deux lapins. Après la troisième injection selon un protocole identique au précédent, les sera ont été prélevés et absorbés contre la lipoprotéine elle-même. Un ELISA a été réalisé avec comme antigène la protéine de fusion lpp-malaria (5*µ*g/puit), comme contrôle la protéine lpp elle-même a été utilisée.

Les résultats sont les suivants :
- pas de réaction avec la lpp
- sérum de lapin immunisé sans adjuvant: 50% de fixation pour la dilution 1/1200
- sérum du lapin immunisé avec adjuvant: 50% de fixation pour la dilution 1/1000.

### Exemple 9 : Présentation d'un épitope T S2B de l'hépatite B

Le principe est de présenter soit la protéine de fusion avec la lipoprotéine à des cellules présentatrices d'antigènes (dans ce cas la souche TA3 qui est une lignée B continue) pendant une demi-heure, puis de laver les cellules et de les incuber avec un hybridome T reconnaissant l'épitope T S2B pendant une nuit. Si la cellule B a présenté correctement l'épitope à l'hybridome, celui-ci sécrète à son tour de l'IL2. Après l'incubation des cellules B activées et de l'hybridome, le surnageant a été prélevé et ajouté à des cellules CTL-L afin de mesurer la prolifération induite par l'IL2 par incorporation de thymidine tritiée. Comme contrôle on a utilisé la lignée B non préincubée ou incubée avec différentes quantités du peptide S2B seul (non fusionné). Une expérience typique a été décrite ci-dessous :
Les cellules TA3 ont été préincubées 1 heure 1/2 (10⁶ cellules/tube) dans la glace.

Elles ont ensuite été lavées 4 fois dans du milieu RPMI + pénicilline et streptomycine.

Elles ont ensuite été préincubées avec différentes concentrations d'antigène:
- (0, 20, 10, 2 *µ*g de la protéine de fusion purifiée par électroélution)
- (0, 30, 6, 3 *µ*g du peptide pur).

Elles ont été transférées dans des puits de plaques microtitres à raison de 5 x 10⁴ cellules par puit dans 200*µ*l.

Après une heure et demie d'incubation, les cellules ont été lavées à nouveau, transférées dans des puits de plaques microtitres à 5 x 10⁴ cellules par puit (en triple exemplaire).

Les cellules de l'hybridome T (souche HB68) ont ensuite été ajoutées à 2 x 10⁴ cellules dans les puits contenant déjà les cellules préincubées.

Les deux types de cellules ont été incubés toute la nuit à 37°C.

Les surnageants ont été prélevés pour un test classique de lymphoprolifération de cellules CTL-L de souris (les autres cellules sont également de souris) en présence de thymidine ³_{H}.

Les résultats suivants ont été obtenus :
Antigène 0: 2.354 cpm
Antigène S2B-lpp 2 µg: 10.393 cpm
Antigène S2B-lpp 10µg: 26.832 cpm
Antigène S2B-lpp 20µg: 44.130 cpm
Antigène S2B 3 µg: 1.438 cpm
Antigène S2B 6 µg: 760 cpm
Antigène S2B 30 µg: 5.035 cpm

### Conclusion

Même pour une concentration élevée de peptide S2B pur (il faut tenir compte du fait que sur une base molaire il y a plus d'antigène S2B présenté que de S2B-lpp), il n'y a pas de bonne présentation à l'hybridome T, tandis que la protéine de fusion lpp présente bien l'épitope.

## Revendications

1. Vecteur recombinant pour l'expression d'une séquence de nucléotides hétérologue dans une bactérie gram-négative, **caractérisé en ce qu'**il comprend, en un site non essentiel pour sa réplication, le gène codant pour une lipoprotéine différente des lipoprotéines de *E. coli* ou une partie de ce gène contenant les éléments nécessaire au contrôle de l'expression de cette lipoprotéine et à son exposition à la surface de la membrane externe de la bactérie gram-négative, de façon telle que la séquence de nucléotides hétérologue est introduite dans le gène ou la partie du gène codant pour la lipoprotéine, dans des conditions permettant l'expression de cette séquence hétérologue et son exposition à la surface de la membrane externe de la bactérie gram-négative.

2. Vecteur recombinant selon la revendication 1, **caractérisé en ce que** la lipoprotéine est une lipoprotéine de structure.

3. Vecteur recombinant selon l'une quelconque des revendications 1 à 2, **caractérisé en ce qu'**il s'agit d'un plasmide.

4. Vecteur recombinant selon l'une quelconque des revendications 1 à 3, **caractérisé en ce que** la lipoprotéine est hétérologue par rapport à la bactérie gram-negative contenant le vecteur.

5. Vecteur recombinant selon l'une quelconque des revendications 1 à 4, **caractérisé en ce que** le gène ou la partie du gène codant pour une lipoprotéine qu'il contient, est le gène codant pour la lipoprotéine majeure de la membrane externe d'une bactérie de genre *Pseudomonas.*

6. Vecteur recombinant selon la revendication 5, **caractérisé en ce que** le gène ou la partie du gène codant pour une lipoprotéine qu'il contient, est le gène codant pour la lipoprotéine majeure de la membrane externe de *Pseudomonas aeruginosa.*

7. Vecteur recombinant selon l'une quelconque des revendications 1 à 6, **caractérisé en ce que** la séquence de nucléotides hétérologue est introduite au niveau de la partie N-terminale du gène ou de la partie du gène codant pour la partie N-terminale de la lipoprotéine mature, de préférence 3 à 4 acides aminés suivant ladite extrémité N-terminale.

8. Vecteur recombinant selon l'une quelconque des revendications 1 à 7, **caractérisé en ce que** le gène ou la partie du gène codant pour une lipoprotéine, est modifié(e) par l'insertion d'un ou plusieurs sites de restriction, par exemple sous la forme d'un polylinker.

9. Vecteur recombinant selon la revendication 8, **caractérisé en ce que** les sites de restriction contenus dans le polylinker sont uniques par rapport aux sites contenus dans le vecteur non recombiné ou dans le gène ou la partie du gène codant pour la lipoprotéine.

10. Vecteur recombinant selon l'une quelconque des revendications 1 à 9, **caractérisé en ce que** l'expression de la séquence hétérologue est constitutive.

11. Vecteur recombinant selon l'une quelconque des revendications 1 à 5, **caractérisé en ce qu'**il s'agit du plasmide pLP134 déposé à la BCCM (LMBP) sous le n° LMBP2916.

12. Vecteur recombinant selon l'une quelconque des revendications 1 à 11, **caractérisé en ce qu'**il s'agit du plasmide pLP135 (tel que décrit dans la Fig. 1 B) ou du plasmide pLP136 (tel que décrit dans la figure 1C).

13. Vecteur recombinant selon l'une quelconque des revendications 1 à 9, **caractérisé en ce que** l'expression de la séquence hétérologue est induite.

14. Vecteur recombinant selon les revendications 13, **caractérisé en ce qu'**il s'agit du vecteur pVUB1 (tel que décrit dans la figure 2B) ou du vecteur pVUB2 (tel que décrit dans la figure 2C).

15. Vecteur recombinant selon l'une quelconque des revendications 1 à 14, **caractérisé en ce que** la séquence de nucléotides hétérologue qu'il contient code pour une séquence d'acides aminés contenant un ou plusieurs déterminants antigéniques ou un ou plusieurs haptènes.

16. Vecteur recombinant selon la revendication 15, **caractérisé en ce que** la séquence de nucléotides hétérologue qu'il contient code pour un épitope de type B et/ou pour un épitope de type T d'antigènes déterminés d'agents pathogènes.

17. Vecteur recombinant selon l'une quelconque des revendications 1 à 14, **caractérisé en ce que** la séquence de nucléotides hétérologue qu'il contient code pour une séquence d'acides aminés d'une chaîne lourde d'un anticorps ou d'une chaîne légère d'un anticorps.

18. Vecteur recombinant selon l'une quelconque des revendications 1 à 14, **caractérisé en ce que** la séquence de nucléotides hétérologue qu'il contient code pour un anticorps constitué par des chaînes lourdes exclusivement.

19. Bactérie gram-négative recombinante **caractérisée en ce qu'**elle est modifiée par un vecteur recombinant selon l'une quelconque des revendications 1 à 18.

20. Bactérie gram-négative recombinante selon la revendication 19, **caractérisée en ce qu'**elle n'exprime pas naturellement la lipoprotéine codée par le gène ou la partie de gène contenue dans le vecteur.

21. Bactérie gram-négative selon la revendication 19, **caractérisée en ce qu'**il s'agit d'une souche de E. *coli.*

22. Bactérie gram-négative selon la revendication 19, **caractérisée en ce qu'**il s'agit d'une souche de *Alcaligenes eutrophus.*

23. Bactérie gram-négative selon l'une quelconque des revendications 19 à 22, **caractérisée en ce que** la séquence de nucléotides hétérologue qu'elle contient, code pour un anticorps ou un fragment d'anticorps.

24. Utilisation de bactéries gram-négative selon la revendication 23, en tant qu'absorbant pour la purification d'antigènes reconnaissant les anticorps ou fragments d'anticorps codés par la séquence nucléotidique hétérologue.

25. Composition immunogène **caractérisée en ce qu'**elle comprend à titre de principe actif une bactérie gram-négative recombinante selon l'une quelconque des revendications 19 à 23.

## Claims

1. Recombinant vector for the expression of a heterologous nucleotide sequence into a gram-negative bacteria, **characterized in that** it comprises, at a site not essential for its replication, the gene encoding a lipoprotein different from the E.coli lipoproteins, or part of this gene which contains the components necessary for controlling the expression of this lipoprotein and for its exposure at the surface of the external cell membrane of the gram-negative bacteria, such that the heterologous nucleotide sequence is introduced into the gene or the part of the gene encoding the lipoprotein, under conditions allowing the expression of this heterologous sequence and its exposure at the surface of the external cell membrane of the gram-negative bacteria.

2. Recombinant vector according to claim 1, **characterized in that** the lipoprotein is a structural lipoprotein.

3. Recombinant vector according to any one of claims 1 to 2, **characterized in that** it is a plasmid.

4. Recombinant vector according to any one of claims 1 to 3, **characterized in that** the structural lipoprotein is heterologous in relation to the gram-negative bacteria containing the vector.

5. Recombinant vector according to any one of claims 1 to 4, **characterized in that** the gene or part of the gene encoding a lipoprotein which it contains, is the gene encoding the major lipoprotein of the external membrane of a bacterium of the genus Pseudomonas.

6. Recombinant vector according to claim 5, **characterized in that** the gene or part of the gene encoding a lipoprotein which it contains, is the gene encoding the major lipoprotein of the external membrane of Pseudomonas aeruginosa.

7. Recombinant vector according to any one of claims 1 to 6, **characterized in that** the heterologous nucleotide sequence is introduced at the level of the N-terminal part of the gene or of the part of the gene encoding the N-terminal domain of the mature lipoprotein, and preferably 3 to 4 amino acids following said N-terminal end.

8. Recombinant vector according to any one of claims 1 to 7, **characterized in that** the gene or part of the gene encoding a lipoprotein is modified by inserting one or more restriction sites, for example in the form of a polylinker.

9. Recombinant vector according to claim 8, **characterized in that** the restriction sites contained in the polylinker are unique in relation to the sites contained in the nonrecombined vector or in the gene or part of the gene encoding the lipoprotein.

10. Recombinant vector according to any one of claims 1 to 9, **characterized in that** the expression of the heterologous sequence is constitutive.

11. Recombinant vector according to any one of claims 1 to 5, **characterized in that** it is the plasmid pLP134 deposited at BCCM (LMBP) under the No. LMBP2916.

12. Recombinant vector according to any one of claims 1 to 11, **characterized in that** it is the plasmid pLPI35 (as described in Fig. 1B) or the plasmid pLPI36 (as described in Fig. 1 C).

13. Recombinant vector according to any one of claims 1 to 9, **characterized in that** the expression of the heterologous sequence is inducible.

14. Recombinant vector according to claim 13, **characterized in that** it is the vector pVUB1 (as described in Fig. 2B) or the vector pVUB2 (as described in Fig. 2C).

15. Recombinant vector according to any one of claims 1 to 14, **characterized in that** the heterologous nucleotide sequence which it contains, encodes an amino acid sequence containing one or more antigenic determinants or one or more haptens.

16. Recombinant vector according to claim 15, **characterized in that** the heterologous nucleotide sequence which it contains, encodes a type B epitope and/or a type T epitope of determined antigens of pathogenic agents.

17. Recombinant vector according to any one of claims 1 to 14, **characterized in that** the heterologous nucleotide sequence which it contains, encodes an amino acid sequence of a heavy chain of an antibody or of a light chain of an antibody.

18. Recombinant vector according to any one of claims 1 to 14, **characterized in that** the heterologous nucleotide sequence which it contains, encodes an antibody consisting exclusively of heavy chains.

19. Recombinant gram-negative bacteria, **characterized in that** it is modified by a recombinant vector according to any one of claims 1 to 18.

20. Recombinant gram-negative bacteria according to claim 19, **characterized in that** it does not express naturally the lipoprotein encoded by the gene or part of the gene contained in the vector.

21. Gram-negative bacteria according to claim 19, **characterized in that** it is a strain of E. coli.

22. Gram-negative bacteria according to claim 19, **characterized in that** it is a strain of Alcaligenes eutrophus.

23. Gram-negative bacteria according to any one of claims 19 to 22, **characterized in that** the heterologous nucleotide sequence which it contains, encodes an antibody or an antibody fragment.

24. Use of host cells according to claim 23, as absorbent for the purification of antigens recognizing the antibodies or antibody fragments encoded by the heterologous nucleotide sequence.

25. Immunogenic composition, **characterized in that** it comprises, as active ingredient, a recombinant gram-negative bacteria according to any one of claims 19 to 23.

## Patentansprüche

1. Rekombinanter Vektor für die Expression einer heterologen Nukleotidsequenz in einem Gram-negativen Bakterium,
**dadurch gekennzeichnet, dass**
er in einer für seine Replikation nicht essenziellen Stelle das Gen umfasst, das für ein von den Lipoproteinen von *E. coli* verschiedenes Lipoprotein kodiert, oder einen Teil dieses Gens, der die für die Kontrolle der Expression dieses Lipoproteins und seine Exposition auf der Oberfläche der äußeren Membran des Gram-negativen Bakteriums notwendigen Elemente enthält, wobei die heterologe Nukleotidsequenz in das Gen oder den Teil des für das Lipoprotein kodierenden Gens in einer Weise eingefügt ist, die die Expression dieser heterologen Sequenz und seine Exposition auf der Oberfläche der äußeren Membran des Gram-negativen Bakteriums erlaubt.

2. Rekombinanter Vektor nach Anspruch 1, **dadurch gekennzeichnet, dass** das Lipoprotein eine Lipoproteinstruktur ist.

3. Rekombinanter Vektor nach irgendeinem der Ansprüche 1 bis 2, **dadurch gekennzeichnet, dass** es sich um ein Plasmid handelt.

4. Rekombinanter Vektor nach irgendeinem der Ansprüche 1 bis 3, **dadurch gekennzeichnet, dass** das Lipoprotein heterolog im Verhältnis zu dem den Vektor enthaltenden Gram-negativen Bakterium ist.

5. Rekombinanter Vektor nach irgendeinem der Ansprüche 1 bis 4, **dadurch gekennzeichnet, dass** das Gen oder der Teil des für ein Lipoprotein kodierenden Gens, den er enthält, das Gen ist, das für das Haupt-Lipoprotein der äußeren Membran eines. Bakteriums der Gattung *Pseudomonas* kodiert.

6. Rekombinanter Vektor nach Anspruch 5, **dadurch gekennzeichnet, dass** das Gen oder der Teil des für ein Lipoprotein kodierenden Gens, den er enthält, das für das Haupt-Lipoprotein der äußeren Membran von *Pseudomonas aeruginosa* kodierende Gen ist.

7. Rekombinanter Vektor nach irgendeinem der Ansprüche 1 bis 6, **dadurch gekennzeichnet, dass** die heterologe Nukleotidsequenz im Bereich des N-terminalen Teils des Gens oder des Teils des Gens, der für den N-terminalen Teil des Gens reifen Lipoproteins kodiert, eingefügt ist, vorzugsweise 3 bis 4 Aminosäuren folgend dem besagten N-terminalen Ende.

8. Rekombinanter Vektor nach irgendeinem der Ansprüche 1 bis 7, **dadurch gekennzeichnet, dass** das Gen oder der Teil des für ein Lipoprotein kodierenden Gens mittels der Insertion einer oder mehrerer Restriktionsstellen modifiziert ist, beispielsweise in Form eines Polylinkers.

9. Rekombinanter Vektor nach Anspruch 8, **dadurch gekennzeichnet, dass** die in dem Polylinker enthaltenen Restriktionsstellen einzig im Verhältnis zu den Stellen sind, die in dem nicht-rekombinierten Vektor oder in dem Gen oder dem Teil des für das Lipoprotein kodierenden Gens enthalten sind.

10. Rekombinanter Vektor nach irgendeinem der Ansprüche 1 bis 9, **dadurch gekennzeichnet, dass** die Expression der heterologen Sequenz konstitutiv ist.

11. Rekombinanter Vektor nach irgendeinem der Ansprüche 1 bis 5, **dadurch gekennzeichnet, dass** es sich um das Plasmid pLPI34 handelt, das bei der BCCM (LMBP) unter der Nr. LMBP2916 hinterlegt ist.

12. Rekombinanter Vektor nach irgendeinem der Ansprüche 1 bis 11, **dadurch gekennzeichnet, dass** es sich um das Plasmid pLPI35 (so wie in der Figur 1B beschrieben) oder um das Plasmid pLPI36 (so wie in der Figur 1C beschrieben) handelt.

13. Rekombinanter Vektor nach irgendeinem der Ansprüche 1 bis 9, **dadurch gekennzeichnet, dass** die Expression der heterologen Sequenz induziert ist.

14. Rekombinanter Vektor nach Anspruch 13, **dadurch gekennzeichnet, dass** es sich um den Vektor pVUB1 (so wie in der Figur 2B beschrieben) oder um den Vektor pVUB2 (so wie in der Figur 2C beschrieben) handelt.

15. Rekombinanter Vektor nach irgendeinem der Ansprüche 1 bis 14, **dadurch gekennzeichnet, dass** die heterologe Nukleotidsequenz, die er enthält, für eine Aminosäuresequenz kodiert, die eine oder mehrere Antigene Determinanten oder ein oder mehrere Haptene enthält.

16. Rekombinanter Vektor nach Anspruch 15, **dadurch gekennzeichnet, dass** die heterologe Nukleotidsequenz, die er enthält, für ein Epitop vom Typ B und/oder für ein Epitop vom Typ T von Antigenen, die von Krankheitserregern bestimmt sind, kodiert.

17. Rekombinanter Vektor nach irgendeinem der Ansprüche 1 bis 14, **dadurch gekennzeichnet, dass** die heterologe Nukleotidsequenz, die er enthält, für eine Aminosäuresequenz einer schweren Kette eines Antikörpers oder einer leichten Kette eines Antikörpers kodiert.

18. Rekombinanter Vektor nach irgendeinem der Ansprüche 1 bis 14, **dadurch gekennzeichnet, dass** die heterologe Nukleotidsequenz, die er enthält, für einen ausschließlich aus schweren Ketten gebildeten Antikörper kodiert.

19. Gram-negatives rekombinantes Bakterium, **dadurch gekennzeichnet, dass** es mittels eines rekombinanten Vektors nach irgendeinem der Ansprüche 1 bis 18 modifiziert ist..

20. Gram-negatives rekombinantes Bakterium nach Anspruch 19, **dadurch gekennzeichnet, dass** es das von dem Gen oder dem in dem Vektor enthaltenen Teil des Gens kodierte Lipoprotein natürlicherweise nicht exprimiert.

21. Gram-negatives Bakterium nach Anspruch 19, **dadurch gekennzeichnet, dass** es sich um einen Stamm von *E. coli* handelt.

22. Gram-negatives Bakterium nach Anspruch 19, **dadurch gekennzeichnet, dass** es sich um einen Stamm von *Alcaligenes eutrophus* handelt.

23. Gram-negatives Bakterium nach irgendeinem der Ansprüche 19 bis 22, **dadurch gekennzeichnet, dass** die heterologe Nukleotidsequenz, die es enthält, für einen Antikörper oder ein Antikörperfragment kodiert.

24. Verwendung Gram-negativer Bakterien nach Anspruch 23 als Absorbens für die Reinigung von Antigenen, die die von der heterologen Nukleotidsequenz kodierten Antikörper oder Antikörperfragmente erkennen.

25. Immunologishe zusammensetzung **dadurch gekennzeichnet dass** es, als aktiv Wirkstoff, ein recombinant es Gram-negatives Bakterium nach irgendeinem der Ansprüche 19 bis 23 umfasst.
